# EUROPEAN PATENT APPLICATION

(11) **EP 4 559 879 A1**
(43) Date of publication of application: **28.05.2025**
(21) Application number: 23211945.3
(22) Date of filing: 24.11.2023
(51) Int. Cl.: C03C 3/083, C03C 3/085, C03C 3/097, C03C 3/118, C03C 4/00, C03C 10/04, C03C 10/16, C03C 3/095, A61C 13/00, A61K 6/833, C03C 10/12

(54) **GLASS-CERAMIC DENTAL BODY, PROCESS FOR PREPARING THE SAME, AND USES THEREOF**

(71) Applicant: Ivoclar Vivadent AG, 9494 Schaan (LI)
(72) Inventor: RAMPF, Markus, 7212 Seewis-Dorf (CH); RITZBERGER, Christian, 9472 Grabs (CH); DITTMER, Marc, 6800 Feldkirch (AT)
(74) Representative: Maiwald GmbH

(57) **Abstract**

A glass-ceramic dental body is provided comprising three consecutive sections: a section A, a section B, and a section C, wherein each one of the sections has a chemical composition that differs from a chemical composition of the other sections. The glass ceramic-dental body may be characterized by a gradient of a mechanical property, an optical property, and/or a thermal property in a direction from section C to section A. Furthermore, a process is provided for preparing a glass-ceramic dental body and a use of a glass-ceramic dental body.

## Description

### TECHNICAL FIELD

The present invention relates to a glass-ceramic dental body such as a glass-ceramic dental blank or a glass-ceramic dental restoration. The present invention further relates to a process for preparing the glass-ceramic dental body and a use of the glass-ceramic dental body in the dental field.

### BACKGROUND OF INVENTION

A dental restoration should ideally have mechanical properties which are comparable to those of natural teeth to provide long durability of the dental restoration while avoiding excessive wear of neighboring tissue and/or natural teeth. Furthermore, it is desirable that a dental restoration resembles the natural appearance of a patient's tooth. Although the appearance of natural teeth may vary, there are some optical characteristics that may be seen as universal for most natural human teeth. A natural tooth usually has a color and/or translucency that changes from the upper incisal or occlusal part of the tooth to its lower dentin part. The upper incisal or occlusal part tooth between those two parts often shows some type of graded change or transition in color and translucency.

Glass-ceramic dental restorations are known in the art. Glass-ceramic dental restorations are often prepared from glass-ceramic dental blanks such as glass-ceramic dental mill blanks or glass-ceramic dental press blanks. A glass-ceramic dental mill blank can be machined, usually using a CAD/CAM process, to a desired shape of the dental restoration. A glass-ceramic dental press blank can be hot pressed in a mold having a desired shape of the dental restoration.

Known glass-ceramic dental bodies, including dental blanks or dental restorations, are often prepared from a single solid glass. The solid glass is subjected to controlled heat to achieve a partial crystallization of the solid glass to produce a glass-ceramic having one or more crystalline phases in its amorphous glassy phase. A glass-ceramic dental body that is prepared from a solid glass typically has uniform mechanical, optical and thermal properties. A glass-ceramic dental body having uniform mechanical, optical, and/or thermal properties is however often not ideal or not acceptable for use in dentistry.

There have been efforts in the art to provide a glass-ceramic dental body by heating different regions of a solid glass at different temperature, e.g., using a gradient furnace. Thereby, crystallization of crystal phases may be varied in different regions of the solid glass. The result may be a glass-ceramic dental body which has one or more non-uniform properties. However, such a preparation process is laborious and glass-ceramic dental bodies obtained by such a process may still have unsatisfactory mechanical, optical or thermal properties.

EP 2 699 521 A1 relates to a process for preparing a glass-ceramic body comprising the steps of providing a basic glass body and subjecting the basic glass body to a thermal treatment whereby a crystalline phase embedded in a glass matrix is formed. The thermal treatment involves a nucleation step followed by several crystallization steps at different temperatures, whereby at least two different crystalline phases are formed. EP 3 974 397 A1 relates to a machinable dental mill block and a method for preparing the same. The method includes the steps of: preparing a block having a predetermined shape from a specific glass composition, and heat-treating the block at a temperature within a range of 760°C to 880°C while applying a temperature gradient to the block in a depth direction.

There is a need in the art for a glass-ceramic dental body having one or more material properties which change in a direction of the body, and ideally in a way that is comparable to a transition of properties in natural teeth and/or in way which allows for providing an improved dental restoration. It is also desirable that such a glass-ceramic dental body is obtainable by a comparatively simple and reproducible process.

### OBJECTS AND SUMMARY OF THE INVENTION

One object of the present invention is to provide a new and improved glass-ceramic dental body. One object of the present invention is to provide a glass-ceramic dental body having one or more material properties that change in a direction of the body. One object of the present invention is to provide a glass-ceramic dental body which does not have a drawback of a prior art glass-ceramic dental body.

One or more of the above objects is solved by the glass-ceramic dental body and the process for preparing the glass-ceramic body according to the embodiments of the present invention.

One aspect of the present invention provides a glass-ceramic dental body comprising three consecutive sections:
a section A,
a section B, and
a section C,
wherein each one of the sections has a chemical composition that differs from a chemical composition of the other sections.

In one preferred embodiment, the glass ceramic-dental body is characterized by one or more gradients of a mechanical property (e.g., a gradient of a biaxial flexural strength and/or a gradient of a fracture toughness (K_{IC})), an optical property (e.g., a gradient of a contrast ratio) and/or a thermal property (e.g., a gradient of a coefficient of the thermal expansion) in a direction from section C to section A.

In one preferred embodiment, a main crystal phase of each one of the sections is the same, and a content of the main crystal phase optionally decreases in a direction from section C to section A. In an alternative preferred embodiment, a main crystal phase of section C is different to a main crystal phase of section A, and optionally a content of the crystal phase, which is the main crystal phase of section C, decreases in a direction from section C to section A, or a content of the crystal phase, which is the main crystal phase of section A, decreases in a direction from section A to section C. In another preferred alternative embodiment, section A is composed of a glass, and section C and section B are composed of a glass-ceramic material.

One finding of the present invention is that a glass-ceramic dental body can be provided that has one or more mechanical, optical and/or thermal properties, like biaxial flexural strength, fracture toughness, translucency or coefficient of thermal expansion, that change in form of a gradient over its consecutive sections. The inventive glass-ceramic dental body can provide or can be used to provide a glass-ceramic dental restoration that has one or more properties that change from one zone to another zone. Thus, the glass-ceramic dental body can provide or can be used for providing dental restorations in which one or more properties are tailored in an advantages way, like in a way that the restoration comes closer to the properties of a natural tooth. For example, a glass-ceramic dental restoration can be provided that has an incisal zone with a higher translucency than its dentin zone. Additionally or alternatively, the thermal properties (e.g., CTE) may be adjusted in a way that different glazings having different thermal properties (e.g., CTE) may be applied to different parts of the restoration.

One aspect of the present invention provides a process for preparing a glass-ceramic dental body according to an embodiment of the present invention. The process comprises the steps of:
- providing two or more powders selected from glass powders, glass-ceramic powders and mixtures thereof;
- preparing a shaped body from the powders; and
- subjecting the shaped body to a heat treatment to obtain a glass-ceramic dental body.

The shaped body typically comprises three consecutive powder sections: a powder section A, a powder section B, and a powder section C, wherein a weight ratio of the two or more powders differs in each one of the powder sections.

It has been found that the inventive glass-ceramic dental body can be obtained in a comparatively simple manner. In particular, the inventive process does not require simultaneously subjecting different regions of a solid glass to different temperatures. Such a process would also be unsuitable for preparing the inventive glass-ceramic dental body because a solid glass has a uniform chemical composition, and therefore cannot be used to obtain different sections having different chemical compositions. Furthermore, the inventors surprisingly found that two or more glass or glass-ceramic powders with different chemical compositions (e.g., glass or glass-ceramic powders which are useful for preparing glass-ceramics with different types and/or contents of crystal phases) can be used for preparing a dense, monolithic glass-ceramic body by heat-treating, and particularly by sintering.

### FIGURE

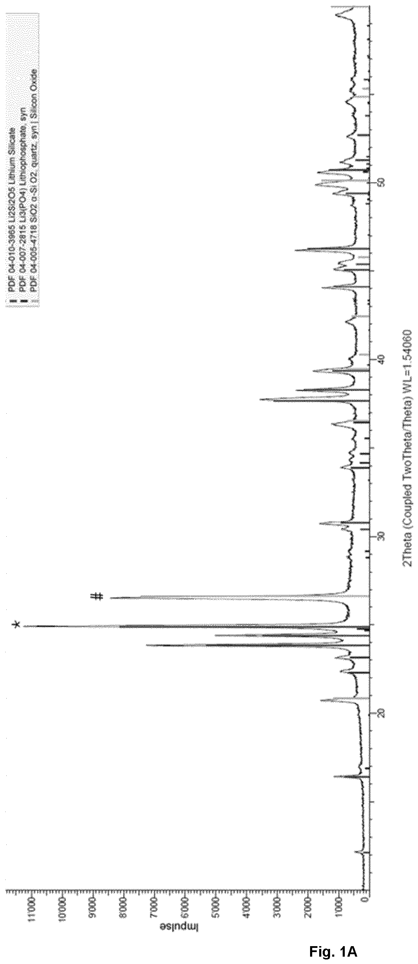

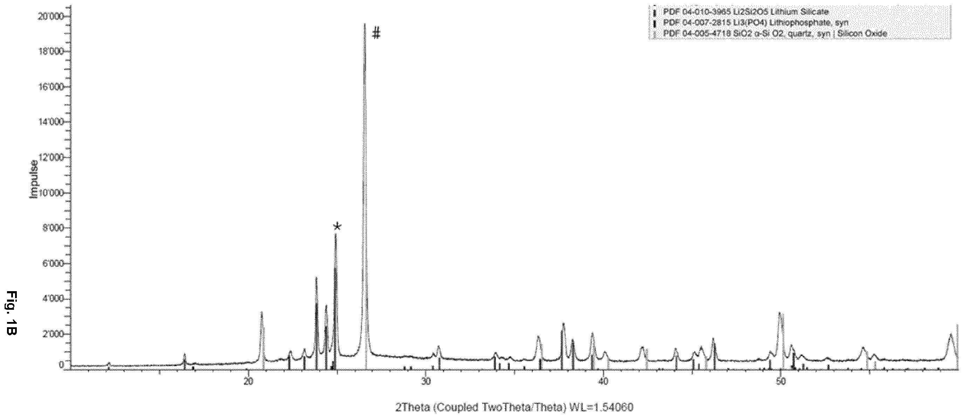

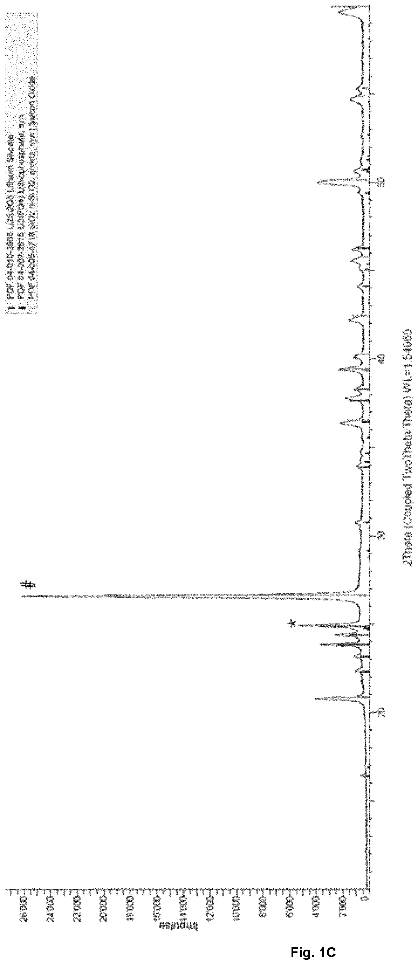

**Fig. 1** shows spectra of a qualitative X-ray diffraction analyses of the different sections of the glass ceramic dental body of example 5. Fig. 1A shows a spectrum of an X-ray diffraction analysis of section C (the bottom layer). Fig. 1B shows a spectrum of an X-ray diffraction analysis of section B (the intermediate layer). Fig. 1C shows a spectrum of an X-ray diffraction analysis of section A (the top layer). The spectra show that the ratio for the peak intensity of lithium disilicate (Li₂Si₂O₅; most prominent peak labelled: *) to α-quartz (most prominent peak labelled: #) changes from the section C (bottom layer) over section B (the intermediate layer) to section A (the top layer). The intensity of the lithium disilicate (Li₂Si₂O₅) decreases from section C (bottom layer) to section A (the top layer) and the intensity of the α-quartz peaks increases from section C (bottom layer) to section A (the top layer). The changing peak intensities indicate that the lithium disilicate (Li₂Si₂O₅) content decreases from section C (bottom layer) to section A (the top layer).

### DEFINITIONS

In the context of the present invention, the following terms have the following meaning:

"Dental body" means a solid, geometrically defined, three-dimensional object of material, like an ingot, block, a disc, or a form of a dental restoration, which is suitable for application in the dental or orthodontic field. A dental body may be, but is not limited to, a dental blank or a dental restoration. A dental blank may be a dental mill blank or a dental press blank.

"Dental mill blank" means a solid, geometrically defined, three-dimensional object of material, like a block or a disc, from which a dental restoration can be machined by, e.g., cutting, milling, grinding, drilling, and the like, typically using a CAD/CAM process.

"Dental press blank" means a solid, geometrically defined, three-dimensional object of material, like a block or an ingot, from which a dental restoration can be formed by hot pressing the blank into a mold having a shape of a dental restoration.

"Dental restoration" as used herein refers to an article that is useful in the dental or orthodontic field for restoring, remodeling, supporting and/or restructuring a tooth or parts thereof or a group of teeth or parts thereof. A dental restoration may be, but is not limited to, a crown, a partial crown, an abutment, an abutment crown, an inlay, an onlay, a veneer, a shell or a bridge.

A "glass-ceramic" dental body means a dental body that is partially or fully composed of a glass-ceramic material. For example, a glass-ceramic dental body which is partially composed of a glass-ceramic material may comprise two sections being composed of a glass-ceramic material and one section being composed of a glass. A glass-ceramic dental body which is fully composed of a glass-ceramic material only comprises sections being composed of a glass-ceramic material. The glass-ceramic dental body typically does not contain a glass-matrix-composite or is typically not composed of a glass-matrix composite. A "glass-matrix composite" in the meaning of the present disclosure is a material which is obtained by addition of crystalline particles to a glass melt or by attaching (e.g., by sintering) a glass material to a crystalline material.

"Glass-ceramic material" means an inorganic, non-metallic solid having a glass phase which surrounds one or more crystal phases. A glass-ceramic material is typically obtained via a controlled nucleation and crystallization of an amorphous base glass. A glass-ceramic material is different from a ceramic material which typically does not contain a glass phase. A "glass" means an inorganic, non-metallic solid which is typically hard, brittle and transparent and essentially free of crystalline regions. It may be described as a thermodynamically unstable frozen melt.

"Consecutive sections" as used herein means sections which are arranged adjacent one after the other. Thus, section A is arranged adjacent to section B, and section B is arranged adjacent to section C.

"Top section" as used herein refers to an outermost section of a glass-ceramic dental blank, like a dental mill blank, that can be used for preparing an incisal or occlusal zone, or a part thereof, in a dental restoration.

"Intermediate section" means a section that is positioned between the top section and the bottom section of a glass-ceramic dental blank, like a dental mill blank. The intermediate section can be used to prepare a transition zone, or a part thereof, of a dental restoration.

"Bottom section" means an outermost section of a glass-ceramic dental blank, , like a dental mill blank, that is located on an opposite side of the glass-ceramic dental blank with respect to the top section. The bottom section can be used to prepare a dentin zone, or a part thereof, of a dental restoration.

The terms "top section", "intermediate section" and "bottom section" are not to be construed in that the glass-ceramic dental blank necessarily has to be positioned (or used) in a specific manner or direction. Any other part that may be additionally present on the outsides of a glass-ceramic dental blank (e.g., a holding pin, a support layer, a protective layer, a printing layer, or a sacrificial layer, like a thin layer of a glass-ceramic material) and that is not suitable or intended to become part of a dental restoration formed from the glass-ceramic dental blank, is not to be understood as a top section, intermediate section or bottom section, or as being part thereof. For example, the glass-ceramic dental blank (e.g., dental mill blank) may be attached on one or more of its outsides to a supporting part, a protective part, and/or a sacrificial part (although this is not necessary). Such parts may be a holding pin, a support layer, a protective layer, a printing layer, or a sacrificial layer, like a thin layer of a glass-ceramic material.

A "layer" means a discrete layer of the glass-ceramic dental body. Discrete layers in a glass-ceramic dental body can be determined by microscopy, like single electron microscopy (SEM).

A "gradient" as used herein means that a property of the glass-ceramic dental body (e.g., a biaxial flexural strength, a fracture toughness (K_{IC}), a contrast ratio, coefficient of thermal expansion (CTE), and the like) increases or decreases (e.g., gradually or in a stepwise manner) in a direction of the glass-ceramic dental body. Thus, a gradient can be defined in that the values, like three or more values, of a property decrease or increase (e.g., gradually or in a stepwise manner) in a direction of the glass-ceramic dental body. A property that decreases "in a direction from section C to section A" means that a value of the property in section C is higher than a value of the property in section B, and the value of the property in section B is higher than a value of the property in section A (i.e., section C > section B > section A). If the property increases "in a direction from section C to section A", the change of the values is the other way round (i.e., section C < section B < section A). A gradient may include that the property changes within a section, like within section B (e.g., stepwise from a layer to another layer or gradually), in a direction from section C to section A.

A "main crystal phase" refers to the crystal phase of the glass-ceramic dental body, or of a section thereof, which has the highest mass fraction of all the crystal phases being present in the glass-ceramic dental body, or of the section thereof. Thus, a main crystal phase can be determined for the glass-ceramic dental body as a whole. In said case, the mass fraction of the crystal phase is based on the total weight of the glass-ceramic dental body. Additionally or alternatively, a main crystal phase can be determined for a section of the glass-ceramic dental body, like a bottom section or a top section as described herein. In said case, the mass fraction of the crystal phase is based on the total weight of the section. The masses of the crystal phases can be determined quantitatively using the Rietveld method. The Rietveld method is well-known in the art.

A "quartz" or "a quartz crystal phase" means a crystal phase selected from the group of α-quartz, α-quartz solid solution, β-quartz solid solution, and mixtures thereof. "Quartz solid solution" means a crystal phase of SiO₂, in which foreign ions are either incorporated into interstitial sites or into lattice sites. These foreign ions may be, but are not limited to, Al³⁺ as well as Mg²⁺, Li⁺ and/or Zn²⁺. For example, Al³⁺ may be present in the solid solution in the same molar concentration as Zn²⁺ and Mg²⁺ taken together.

Where the term "comprising" is used herein, it does not exclude that further non-specified elements are present. Where the term "essentially consisting of" is used herein, it does not exclude that further non-specified elements are present that are not materially affecting the essential characteristics of the defined subject-matter. When e.g., a section or a layer is defined by its chemical composition, the section or layer may contain unavoidable trace impurities in a sum of < 0.2 wt.%, even if this is not explicitly defined. For the purposes of the present invention, the terms "essentially consisting of" and "consisting of" are considered to be specific embodiments of the term "comprising of". Whenever the terms "including" or "having" are used, these terms are meant to be equivalent to "comprising" as defined above.

The term "obtained" does not necessarily mean to indicate that, e.g., an embodiment must be obtained by, e.g., a sequence of steps following the term "obtained" even though such a limited understanding is always included by the term "obtained" as a preferred embodiment.

Numbers defined herein are rounded to their last digit and are meant to encompass the range of rounding values according to established rounding rules. For example, the value 3 is meant to encompass the values in the range of 2.5 to 3.4, the value 1.5 is meant to encompass the values in the range of 1.46 to 1.54, and so on.

In the following, the present invention is described in more detail.

### DETAILED DESCRIPTION OF THE INVENTION

### I. Glass-ceramic dental body

The present invention provides a glass-ceramic dental body comprising three consecutive sections:
a section A,
a section B, and
a section C,
wherein each one of the sections has a chemical composition that differs from a chemical composition of the other sections.

In one preferred embodiment, the glass ceramic-dental body is characterized by one or more gradients of a mechanical property, an optical property, and/or a thermal property in a direction from section C to section A.

The glass-ceramic dental body may be further defined by one or more specific gradients of mechanical, optical, and/or thermal properties. Alternatively or additionally, the glass-ceramic dental body may be defined by its crystal phases, by its chemical composition, and/or its structure and form.

### 1. Gradients

In one preferred embodiment, the glass ceramic-dental body is characterized by one or more gradients of a mechanical property, an optical property, and/or a thermal property in a direction from section C to section A. The one or more gradients may be a gradient of one or more mechanical properties such as, but not limited, to a gradient of a biaxial flexural strength, a gradient of a fracture toughness (K_{IC}) and/or a gradient of machinability. The one or more gradients may be a gradient of one or more optical properties such as, but not limited to, a gradient of a contrast ratio. The one or more gradients may be a gradient of one or more thermal properties such as, but not limited to, a gradient of a coefficient of the thermal expansion (CTE).

### 1.1 Gradient of a mechanical property

The one or more gradients may be a gradient of a biaxial flexural strength, wherein the biaxial flexural strength decreases from section C to section A. The biaxial flexural strength can be determined according to DIN EN ISO 6872 (DIN EN ISO 6872:2019). A test body of each one of the sections can be obtained from the respective section of the glass-ceramic dental body, e.g., by milling, cutting, and/or sawing with diamond-coated tools. When a section has a dimension which is not suitable for preparing an adequate test body for determining the biaxial flexural strength (e.g., according to DIN EN ISO 6872, and particularly according to DIN EN ISO 6872:2019), then an adequate test body can be prepared from suitable raw materials after analysis of the crystal phases and/or chemical composition of the respective section.

Section C may have a biaxial flexural strength of at least 150 MPa, at least 170 MPa, at least 200 MPa, at least 250 MPa, at least 300 MPa or at least 350 MPa. Section C may have a biaxial flexural strength of at most 550 MPa, at most 500 MPa, at most 475 MPa, at most 450 MPa, at most 350 MPa, at most 300 MPa, or at most 250 MPa. Section C may have a biaxial flexural strength in a range of 150 to 550 MPa, in a range of 175 to 500 MPa, in a range of 200 to 475 MPa, in a range of 250 to 450 MPa, in a range of 350 to 475 MPa, in a range of 250 to 350 MPa or in a range of 150 to 250 MPa. A biaxial flexural strength of at least 200 MPa is typically preferred for section C since such a biaxial flexural strength is specifically suitable for providing a dentin zone of a dental restoration with a good mechanical strength.

Section C may have a biaxial flexural strength that is at least 25 MPa, at least 60 MPa, at least 100 MPa, at least 150 MPa, at least 175 MPa or at least 200 MPa, higher than a biaxial flexural strength of section A. Section C may have a biaxial flexural strength that is at most 400 MPa, at most 350 MPa, at most 300 MPa, or at most 250 MPa, higher than a biaxial flexural strength of section A. Section C may have a biaxial flexural strength that is higher by a value in a range of 25 to 400 MPa, 60 to 400 MPa, in a range of 100 to 350 MPa, in a range of 150 to 300 MPa, in a range of 175 to 250 MPa, in a range of 200 to 400 MPa, or in a range of 60 to 200 MPa, than a biaxial flexural strength of section A.

Section B may have a biaxial flexural strength of at least 150 MPa, at least 200 MPa, at least 250 MPa, at least 300 MPa or at least 350 MPa. Section B may have a biaxial flexural strength of at most 500 MPa, at most 450 MPa, at most 350 MPa, at most 300 MPa, or at most 250 MPa. Section B may have a biaxial flexural strength in a range of 150 to 500 MPa, 200 to 450 MPa, 300 to 450 MPa, 350 to 450 MPa, 200 to 400 MPa, 250 to 400 MPa, or 150 to 250 MPa.

Section A may have a biaxial flexural strength of at least 75 MPa, at least 100 MPa, at least 150 MPa or at least 200 MPa. Section A may have a biaxial flexural strength of at most 350 MPa, at most 300 MPa, at most 250 MPa or at most 200 MPa. Section A may have a biaxial flexural strength in a range of 75 to 350 MPa, in a range of 100 to 300 MPa, in a range of 150 to 250 MPa, in a range of 200 to 300 MPa, in a range of 100 to 200 MPa, or in a range of 75 to 200 MPa.

In one embodiment, section C has a biaxial flexural strength in a range of 150 to 250 MPa and section A has a biaxial flexural strength in a range of 75 to 200 MPa. In one embodiment, section C has a biaxial flexural strength in a range of 250 to 350 MPa and section A has a biaxial flexural strength in a range of 100 to 250 MPa. In one embodiment, section C has a biaxial flexural strength in a range of 350 to 475 MPa and section A has a biaxial flexural strength in a range of 150 to 300 MPa. In one embodiment, section C has a biaxial flexural strength in a range of 350 to 475 MPa and section A has a biaxial flexural strength in a range of 100 to 200 MPa. In one embodiment, section C has a biaxial flexural strength in a range of 250 to 355 MPa and section A has a biaxial flexural strength in a range of 220 to 320 MPa. In view of the gradient of the biaxial flexural strength, it is to be understood that section B has a biaxial flexural strength of a value which is between the values of section C and section A.

The one or more gradients may be a gradient of a fracture toughness (K_{IC}), wherein the fracture toughness (K_{IC}) decreases from section C to section A. The fracture toughness (K_{IC}) can be determined according to the Single Edge V-Notched Beam (SEVNB) method according to DIN EN ISO 6872, and particularly DIN EN ISO 6872:2015. A test body of each one of the sections can be obtained from the respective section of the glass-ceramic dental body, e.g., by milling, cutting, and/or sawing with diamond-coated tools. When a section has a dimension which is not suitable for preparing an adequate test body for determining the fracture toughness (K_{IC}) (e.g., according to DIN EN ISO 6872, and particularly DIN EN ISO 6872:2015), then an adequate test body can be prepared from suitable raw materials after analysis of the crystal phases and/or chemical composition of the respective section.

Section C may have a fracture toughness (K_{IC}) of at least 1.4 MPa*m^{-1/2}, 1.6 MPa*m^{-1/2}, at least 1.8 MPa*m^{-1/2}, 2.0 MPa*m^{-1/2}, at least 2.2 MPa*m^{-1/2}, or at least 2.6 MPa*m^{-1/2}. Section C may have a fracture toughness (K_{IC}) of at most 3.4 MPa*m^{-1/2}, at most 3.0 MPa*m^{-1/2}, at most 2.8 MPa*m^{-1/2}, at most 2.6 MPa*m^{-1/2}, at most 2.4 MPa*m^{-1/2}, or at most 2.2 MPa*m^{-1/2}. Section C may have a fracture toughness (K_{IC}) in a range of 1.4 to 3.4 MPa*m^{-1/2}, preferably in a range of 1.6 to 2.8 MPa*m^{-1/2}, and more preferably in a range of at least 1.8 to 2.6 MPa*m^{-1/2}. Section C may have a fracture toughness (K_{IC}) in a range of 1.4 to 2.0 MPa*m^{-1/2}, in a range of 1.6 to 2.2 MPa*m^{-1/2}, in a range of 2.0 to 2.6 MPa*m^{-1/2}, or in a range of 2.6 to 3.4 MPa*m^{-1/2}. A fracture toughness (K_{IC}) of at least 2.0 MPa*m^{-1/2} is typically preferred for section C as such a fracture toughness is specifically suitable for providing a dentin zone of a dental restoration with a good mechanical strength.

Section C may have a fracture toughness (K_{IC}) that is at least 0.4 MPa*m^{-1/2}, at least 0.6 MPa*m^{-1/2}, at least 0.8 MPa*m^{-1/2}, at least 1.0 MPa*m^{-1/2}, or at least 1.2 MPa*m^{-1/2}, higher than a fracture toughness (K_{IC}) of section A. Section C may have a fracture toughness (K_{IC}) that is at most 2.0 MPa*m^{-1/2}, at most 1.8 MPa*m^{-1/2}, at most 1.4 MPa*m^{-1/2}, at most 1.2 MPa*m^{-1/2}, or at most 0.8 MPa*m^{-1/2}, higher than a fracture toughness (K_{IC}) of section A. Section C may have a fracture toughness (K_{IC}) that is higher by a value in a range of 0.4 to 2.0 MPa*m^{-1/2}, in a range of 0.6 to 2.0 MPa*m^{-1/2}, in a range of 0.8 to 1.8 MPa*m^{-1/2}, in a range of 1.0 to 1.8 MPa*m^{-1/2}, in a range of 1.2 to 1.8 MPa*m^{-1/2}, in a range of 0.4 to 1.2 MPa*m^{-1/2}, or in a range of 0.4 to 1.0 MPa*m^{-1/2}, than a fracture toughness (K_{IC}) of section A.

Section B may have a fracture toughness (K_{IC}) of at least 1.2 MPa*m^{-1/2}, 1.4 MPa*m^{-1/2}, at least 1.6 MPa*m^{-1/2}, at least 1.8 MPa*m^{-1/2}, or at least 2.4 MPa*m^{-1/2}. Section B may have a fracture toughness (K_{IC}) of at most 3.2 MPa*m^{-1/2}, at most 2.8 MPa*m^{-1/2}, at most 2.6 MPa*m^{-1/2}, at most 2.4 MPa*m^{-1/2}, or at most 2.2 MPa*m^{-1/2}. Section B may have a fracture toughness (K_{IC}) in a range of 1.2 to 3.2 MPa*m^{-1/2}, in a range of 1.4 to 2.6 MPa*m^{-1/2}, in a range of at least 1.6 to 2.4 MPa*m^{-1/2}, in a range of at least 1.6 to 2.2 MPa*m^{-1/2}, or in a range of at least 1.2 to 2.2 MPa*m^{-1/2}, or in a range of at least 2.4 to 3.2 MPa*m^{-1/2}.

Section A may have a fracture toughness (K_{IC}) of at least 0.5 MPa*m^{-1/2}, at least 0.7 MPa*m^{-1/2}, at least 1.2 MPa*m^{-1/2}, at least 1.2 MPa*m^{-1/2}, or at least 1.4 MPa*m^{-1/2}, or at least 2.4 MPa*m^{-1/2}. Section A may have a fracture toughness (K_{IC}) of at most 3.0 MPa*m^{-1/2}, at most 2.2 MPa*m^{-1/2}, at most 2.0 MPa*m^{-1/2}, at most 1.8 MPa*m^{-1/2}, at most 1.6 MPa*m^{-1/2}, at most 1.2 MPa*m^{-1/2} or at most 1.0 MPa*m^{-1/2}. Section A may have a fracture toughness (K_{IC}) in a range of 0.5 to 2.2 MPa*m^{-1/2}, preferably in a range of 0.7 to 2.0 MPa*m^{-1/2}, and more preferably in a range of at least 0.7 to 1.8 MPa*m^{-1/2}. Section A may have a fracture toughness (K_{IC}) in a range of 0.5 to 1.2 MPa*m^{-1/2}, in a range of 0.7 to 1.4 MPa*m^{-1/2}, in a range of 1.2 to 1.8 MPa*m^{-1/2}, or in a range of 1.4 to 2.2 MPa*m^{-1/2}, or in a range of 2.4 to 3.0 MPa*m^{-1/2}.

In one embodiment, section C has a fracture toughness (K_{IC}) in a range of 1.2 to 2.0 MPa*m^{-1/2} and section A has a fracture toughness (K_{IC}) in a range of 0.5 to 1.2 MPa*m^{-1/2}. In one embodiment, section C has a fracture toughness (K_{IC}) in a range of 2.0 to 2.6 MPa*m^{-1/2} and section A has a fracture toughness (K_{IC}) in a range of 1.4 to 2.2 MPa*m^{-1/2}. In one embodiment, section C has a fracture toughness (K_{IC}) in a range of 2.2 to 3.0 MPa*m^{-1/2} and section A has a fracture toughness (K_{IC}) in a range of 0.5 to 1.4 MPa*m^{-1/2}. In one embodiment, section C has a fracture toughness (K_{IC}) in a range of 2.6 to 3.4 MPa*m^{-1/2} and section A has a fracture toughness (K_{IC}) in a range of 2.4 to 3.0 MPa*m^{-1/2}. In view of the gradient of the fracture toughness (K_{IC}), it is to be understood that section B has a fracture toughness (K_{IC}) of a value which is between the values of section C and section A.

The one or more gradients may be a gradient of a machinability, wherein the machinability increases from section C to section A.

### 1.2 Gradient of an optical property

The one or more gradients may be a gradient of a contrast ratio, wherein the contrast ratio decreases from section C to section A. The contrast ratio can be determined according to BS 5612, and particularly BS 5612:1978. The contrast ratio is typically determined using test bodies having a thickness of 2 mm ± 0.02 mm. The test bodies can be prepared from the different sections of the glass-ceramic dental body (e.g., by milling, cutting, and/or sawing with diamond-coated tools). Before carrying out the measurement, the surface of the test bodies may be ground and polished as described herein in the section "Measuring methods". The contrast ratio relates to the ratio of illuminance (Y) of a material when placed on a black background (Yb) to the illuminance of the same material when placed over a white background (Yw) (CR = Yb/Yw). The contrast ratio can be used to characterize the translucency of a material, i.e. the light transmission of a material expressed as the ratio of transmitted to incident light intensity. A contrast ratio of close to 0% may indicate that a given material is almost fully transparent, while a contrast ratio of 100% may indicate that a material is fully opaque. When a section has a dimension which is not suitable for preparing an adequate test body for determining the contrast ratio (K_{IC}) (e.g., according to BS 5612, and particularly BS 5612:1978), then an adequate test body can be prepared from suitable raw materials after analysis of the crystal phases and/or chemical composition of the respective section.

Section C may have a contrast ratio of at least 62%, at least 65%, at least 75%, at least 80%, or at least 85%. Section C may have a contrast ratio of at most 94%, at most 92%, at most 85%, at most 75% or at most 72%. Section C may have a contrast ratio in a range of 62 to 94%, in a range of 65 to 92%, in a range of 75 to 85%, in a range of 80 to 94%, in a range of 85 to 92%, in a range of 62 to 75% or in a range of 65 to 72%.

Section C may have a contrast ratio that is higher than a contrast ratio of section A by at least 10 percent points, at least 15 percent points, at least 20 percent points, at least 36 percent points, or at least 42 percent points. Section C may have a contrast ratio that is higher than a contrast ratio of section A by at most 56 percent points, at most 52 percent points, at most 35 percent points, at most 30 percent points, or at most 26 percent points. Section C may have a contrast ratio that is higher than a contrast ratio of section A by in range of 10 to 56 percent points, in a range of 15 to 52 percent points, in a range of 36 to 56 percent points, in a range of 42 to 52 percent points, in a range of 10 to 35 percent points, in a range of 15 to 30 percent points, or in a range of 20 to 26 percent points.

Section B may have a contrast ratio of at least 40%, at least 45%, at least 50%, at least 55%, or at least 60%. Section B may have a contrast ratio of at most 90%, at most 85%, at most 80%, at most 75%, or at most 65%. Section B may have a contrast ratio in a range of 40 to 90%, in a range of 45 to 85%, in a range of 50 to 80%, in a range of 55 to 75%, in a range of 60 to 75% or in a range of 55 to 65%.

Section A may have a contrast ratio of at least 14%, at least 18%, at least 55%, at least 58%, or at least 60%. Section A may have a contrast ratio of at most 80%, at most 74%, at most 70%, at most 65%, at most 30%, or at most 25%. Section A may have a contrast ratio in a range of 14 to 80%, in a range of 18 to 74%, in a range of 55 to 80%, in a range of 58 to 74%, in a range of 60 to 70%, in a range of 55 to 65%, in a range of 14 to 30% or in a range of 18 to 25%.

In one embodiment, section C has a contrast ratio in a range of 80 to 94% (e.g., in a range of 85 to 92%) and section A has a contrast ratio in a range of 58 to 74% (e.g., 60 to 70%). In one embodiment, section C has a contrast ratio in a range of 62 to 75% (e.g., in a range of 65 to 72%) and section A has a contrast ratio in a range of 14 to 30% (e.g., 18 to 25%). In one embodiment, section C has a contrast ratio in a range of 75 to 85% and section A has a contrast ratio in a range of 55 to 65%.

### 1.3 Gradient of a thermal property

The one or more gradients may be a gradient of a coefficient of the thermal expansion (CTE), wherein the coefficient of the thermal expansion decreases or increases from section C to section A. The coefficient of the thermal expansion can be determined using a dilatometer according to DIN EN ISO 6872, and particularly DIN EN ISO 6872:2015. A test body can be obtained from each one of the sections of the glass-ceramic dental body by milling, cutting, and/or sawing the respective section of the glass-ceramic dental body with diamond-coated tools. When a section has a dimension which is not suitable for preparing an adequate test body for determining the coefficient of the thermal expansion (e.g., according to DIN EN ISO 6872, and particularly DIN EN ISO 6872:2015), then an adequate test body can be prepared from suitable raw materials after analysis of the crystal phases and/or chemical composition of the respective section.

Each one of the sections may have a coefficient of the thermal expansion in a range of 5 to 14 ppm/K. For example, section C may have a coefficient of the thermal expansion of in a range of 7 to 13 ppm/K or in a range of 8 to 12 ppm/K. Section B may have a coefficient of the thermal expansion of in a range of 6 to 13 ppm/K or in a range of 7 to 12 ppm/K. Section A may have a coefficient of the thermal expansion of in a range of 5 to 14 ppm/K or in a range of 6 to 13 ppm/K.

Section C may have a coefficient of the thermal expansion which differs from a coefficient of the thermal expansion of section A by at least 1 ppm/K. Section C may have a coefficient of the thermal expansion which differs from a coefficient of the thermal expansion of section A by at most 4 ppm/K or at most 3 ppm/K. Section C may have a coefficient of the thermal expansion which differs from a coefficient of the thermal expansion of section A by in a range of 1 to 4 ppm/K or in a range of 1 to 3 ppm/K. In one embodiment, section C has a coefficient of the thermal expansion in a range of 7 to 13 ppm/K (e.g., in a range of 8 to 12 ppm/K) and section A has a coefficient of the thermal expansion in a range of 5 to 14 ppm/K (e.g., in a range of 6 to 13 ppm/K), and section C has a coefficient of the thermal expansion which differs from a coefficient of the thermal expansion of section A by in a range of 1 to 4 ppm/K (e.g., in a range of 1 to 3 ppm/K).

In the following, an embodiment of the present invention is described which is referred to herein as "embodiment CTE-1". In embodiment CTE-1, the gradient is a gradient of a coefficient of the thermal expansion (CTE), wherein the coefficient of the thermal expansion decreases from section C to section A.

In embodiment CTE-1, section C may have a coefficient of the thermal expansion of at least 6.6 ppm/K, at least 7.6 ppm/K, or at least 8.2 ppm/K. In embodiment CTE-1, section C may have a coefficient of the thermal expansion of at most 10.6 ppm/K, at most 9.6 ppm/K, or at most 9.0 ppm/K. In embodiment CTE-1, section C may have a coefficient of the thermal expansion of in a range of 6.6 to 10.6 ppm/K, in a range of 7.6 to 9.6 ppm/K, or in a range of 8.2 to 9.0 ppm/K.

In embodiment CTE-1, section C may have a coefficient of the thermal expansion which is higher than a coefficient of the thermal expansion of section A by at least 1.0 ppm/K, at least 1.8 ppm/K, or at least 2.2 ppm/K. In embodiment CTE-1, section C may have a coefficient of the thermal expansion which is higher than a coefficient of the thermal expansion of section A by at most 4.0 ppm/K, at most 3.2 ppm/K, or at most 2.8 ppm/K. In embodiment CTE-1, section C may have a coefficient of the thermal expansion which is higher than a coefficient of the thermal expansion of section A by in a range of 1.0 to 4.0 ppm/K, in a range of 1.8 to 3.2 ppm/K, or in a range of 2.2 to 2.8 ppm/K.

In embodiment CTE-1, section B may have a coefficient of the thermal expansion of at least 5.2 ppm/K, at least 6.2 ppm/K, or at least 7.0 ppm/K. In embodiment CTE-1, section B may have a coefficient of the thermal expansion of at most 9.2 ppm/K, at most 8.2 ppm/K, or at most 7.6 ppm/K. In embodiment CTE-1, section B may have a coefficient of the thermal expansion of in a range of 5.2 to 9.2 ppm/K, in a range of 6.2 to 8.2 ppm/K, or in a range of 7.0 to 7.6 ppm/K.

In embodiment CTE-1, section A may have a coefficient of the thermal expansion of at least 4.6 ppm/K, at least 5.2 ppm/K, or at least 5.8 ppm/K. In embodiment CTE-1, section A may have a coefficient of the thermal expansion of at most 8.2 ppm/K, at most 7.2 ppm/K, or at most 6.6 ppm/K. In embodiment CTE-1, section A may have a coefficient of the thermal expansion of in a range of 4.6 to 8.2 ppm/K, in a range of 5.2 to 7.2 ppm/K, or in a range of 5.8 to 6.6 ppm/K.

In one more specific embodiment of embodiment CTE-1, section C has a coefficient of the thermal expansion in a range of 6.6 to 10.6 ppm/K (e.g., in a range of 7.6 to 9.6 ppm/K) and section A has a coefficient of the thermal expansion in a range of 4.6 to 8.2 ppm/K (e.g., in a range of 5.2 to 7.2 ppm/K), and section C has a coefficient of the thermal expansion which is higher than a coefficient of the thermal expansion of section A by in a range of 1.0 to 4.0 ppm/K (e.g., in a range of 1.8 to 3.2 ppm/K).

In the following, an embodiment of the present invention is described which is referred to herein as "embodiment CTE-2". In embodiment CTE-2, the gradient is a gradient of a coefficient of the thermal expansion (CTE), wherein the coefficient of the thermal expansion increases from section C to section A.

In embodiment CTE-2, section C may have a coefficient of the thermal expansion of at least 9.2 ppm/K, at least 10.2 ppm/K, or at least 10.6 ppm/K. In embodiment CTE-2, section C may have a coefficient of the thermal expansion of at most 13.2 ppm/K, at most 12.2 ppm/K, or at most 11.8 ppm/K. In embodiment CTE-2, section C may have a coefficient of the thermal expansion of in a range of 9.2 to 13.2 ppm/K, in a range of 10.2 to 12.2 ppm/K, or in a range of 10.6 to 11.8 ppm/K.

In embodiment CTE-2, section C may have a coefficient of the thermal expansion which is lower than a coefficient of the thermal expansion of section A by at least 0.8 ppm/K, at least 1.0 ppm/K, or at least 1.2 ppm/K. In embodiment CTE-2, section C may have a coefficient of the thermal expansion which is lower than a coefficient of the thermal expansion of section A by at most 2.5 ppm/K, at most 2.0 ppm/K, or at most 1.8 ppm/K. In embodiment CTE-2, section C may have a coefficient of the thermal expansion which is lower than a coefficient of the thermal expansion of section A by in a range of 0.8 to 2.5 ppm/K, in a range of 1.0 to 2.0 ppm/K, or in a range of 1.2 to 1.8 ppm/K.

In embodiment CTE-2, section B may have a coefficient of the thermal expansion of at least 10.0 ppm/K, at least 11.0 ppm/K, or at least 10.4 ppm/K. In embodiment CTE-1, section B may have a coefficient of the thermal expansion of at most 14.0 ppm/K, at most 13.0 ppm/K, or at most 12.6 ppm/K. In embodiment CTE-1, section B may have a coefficient of the thermal expansion of in a range of 10.0 to 14.0 ppm/K, in a range of 11.0 to 13.0 ppm/K, or in a range of 10.4 to 12.6 ppm/K.

In embodiment CTE-1, section A may have a coefficient of the thermal expansion of at least 10.7 ppm/K, at least 11.7 ppm/K, or at least 12.1 ppm/K. In embodiment CTE-1, section A may have a coefficient of the thermal expansion of at most 14.4 ppm/K, at most 13.7 ppm/K, or at most 13.3 ppm/K. In embodiment CTE-1, section A may have a coefficient of the thermal expansion of in a range of 10.7 to 14.4 ppm/K, in a range of 11.7 to 13.7 ppm/K, or in a range of 12.1 to 13.3 ppm/K.

In one more specific embodiment of embodiment CTE-1, section C has a coefficient of the thermal expansion in a range of 9.2 to 13.2 ppm/K (e.g., in a range of 10.2 to 12.2 ppm/K) and section A has a coefficient of the thermal expansion in a range of 10.7 to 14.4 ppm/K (e.g., in a range of 11.7 to 13.7 ppm/K), and section C has a coefficient of the thermal expansion which is lower than a coefficient of the thermal expansion of section A by in a range of 0.8 to 2.5 ppm/K (e.g., in a range of 1.0 to 2.0 ppm/K).

### 1.4 Further characteristics of the gradient(s)

It is also possible, and sometimes preferred, that the glass-ceramic dental body is characterized by a combination of gradients of one or more material properties as defined herein. In one embodiment, the glass-ceramic dental body is characterized by a combination of gradients of two or more mechanical properties, like a combination of the gradient of a biaxial flexural strength and the gradient of the fracture toughness (K_{IC}). In one embodiment, the glass-ceramic dental body is characterized by a combination of a gradient of one or more mechanical property and a gradient of one or more optical properties, like a combination of the gradient of a contrast ratio and the gradient of a biaxial flexural strength and/or the gradient of the fracture toughness (K_{IC}).

The one or more gradients may be characterized by a gradual change or a stepwise change of the respective property. A gradual change is typically achieved when the glass-ceramic dental body is composed of a section B having a chemical composition that gradually changes in a direction from section C to section A. A stepwise change is typically achieved when the glass-ceramic dental body is composed of a multi-layer structure. The structure of the glass-ceramic dental body is further described in other sections of this disclosure.

Additionally or alternatively to the one or more gradients, the glass-ceramic dental body may be characterized by its crystal phases. This is described in more detail in the following section.

### 2. Crystal phases

The glass-ceramic dental body has a main crystal phase. The main crystal phase may be, but is not limited to, lithium disilicate, a quartz (e.g., α-quartz, α-quartz solid solution or a β-quartz solid solution), or a stoichiometric or non-stoichiometric lithium alumosilicate (e.g., spodumene or spodumene solid solution).

The main crystal phase of the glass-ceramic dental body is typically not lithium metasilicate. In one embodiment, the glass-ceramic dental body does not contain a lithium metasilicate crystal phase in a mass fraction of more than 5 wt.% or more than 3 wt.%, based on the total weight of the glass-ceramic dental body.

The sections of the glass-ceramic dental body may comprise different crystal phases. In one embodiment of the present invention, section A may comprise one or more crystal phases which are different to a crystal phase in section C (or vice versa). Crystal phases in section C, section B, and/or section A may be, but are not limited to, lithium disilicate, lithium silicate, apatite (e.g., fluorapatite), one or more quartz crystal phases (e.g., α-quartz, α-quartz solid solution and/or β-quartz solid solution), stoichiometric lithium alumosilicates (e.g., eucryptite, spodumene, spodumene solid solution, and/or petalite), non-stoichiometric lithium alumosilicate phases (e.g., Li₂O·Al₂O₃·7.5SiO₂ with keatite crystal structure), cristobalite, lithium phosphate, a magnesium silicate (e.g., enstatite), an alkalizirconium silicate (e.g., sogdianite or zektzerite), diopside or wollastonite.

Each one of the sections may comprise at least one (e.g., one, two or three) crystal phase in a content which differs from a content of the respective crystal phase in the other sections. This includes that a section may comprise the respective crystal phase in 0 wt.%.

Each one of the sections of the glass-ceramic dental body may be composed of a glass-ceramic material. Thus, in certain embodiments, each one of the sections of the glass-ceramic dental body is composed of a glass-ceramic material. Alternatively, section A may be composed of a glass. This can be understood in that section A essentially does not contain a crystal phase (0 wt.% of a crystal phase). Section A may be composed of a glass, and section B and section C may be composed of a glass-ceramic material. Thus, in certain embodiments, section A is composed of a glass, and section B and section C are composed of a glass-ceramic material.

Each section of the glass-ceramic dental body, which is composed of a glass-ceramic material, can be defined by a main crystal phase, and optionally one or more minor crystal phases (i.e., one or more crystal phases that are present in a section in a content being less than the content of the main crystal phase).

The glass-ceramic dental body may be defined by the main crystal phase of its section C. The main crystal phase of section C may be lithium disilicate, a quartz (e.g., α-quartz, α- or β-quartz solid solution), a stoichiometric lithium alumosilicate (e.g., eucryptite, spodumene, spodumene solid solution, or petalite) or non-stoichiometric lithium alumosilicate (e.g., Li₂O·Al₂O₃·7.5SiO₂ with keatite crystal structure). In one embodiment, the main crystal phase of section C is lithium disilicate or a quartz (e.g., α-quartz, α- or β-quartz solid solution). The main crystal phase of section C is typically lithium disilicate (although this is not necessary). The main crystal phase of section C is typically not lithium metasilicate. The main crystal phase of section C may be present in an amount in a range of 20 to 80 wt.%, based on the total weight of section C.

The main crystal phase of section C may be the only crystal phase of section C. However, section C typically comprises one or more minor crystal phases. The one or more minor crystal phase may be, but is not limited to, lithium disilicate, lithium silicate, apatite (e.g., fluorapatite), one or more quartz crystal phases (e.g., α-quartz, α-quartz solid solution and/or β-quartz solid solution), a stoichiometric lithium alumosilicate (e.g., eucryptite, spodumene, spodumene solid solution, and/or petalite), a non-stoichiometric lithium alumosilicate phase (e.g. Li₂O·Al₂O₃·7.5SiO₂ with keatite crystal structure), lithium phosphate, a magnesium silicate (e.g., enstatite), an alkalizirconium silicate (e.g. sogdianite or zektzerite), diopside or wollastonite.

The glass-ceramic dental body may be defined by the main crystal phase of its section A. The main crystal phase of section A may be lithium disilicate, an apatite, a quartz (e.g., α-quartz, α-quartz solid solution or β-quartz solid solution), or a lithium alumosilicate (LAS). The apatite may be, but is not limited to, a fluorapatite. The lithium alumosilicate (LAS) may be a stoichiometric lithium alumosilicate such as eucryptite (LiAlSiO₄), spodumene (LiAl(SiO₃)₂), spodumene solid solution, or petalite (LiAlSi₄O₁₀) or a non-stoichiometric lithium alumosilicate phase such as Li₂O·Al₂O₃·7.5SiO₂.

The main crystal phase of section A may be the only crystal phase of section A. However, it is also possible that section A comprises one or more minor crystal phases. The one or more minor crystal phases may be, but is not limited to, lithium disilicate, lithium silicate, apatite (e.g., fluorapatite), one or more quartz crystal phases (e.g., α-quartz, α-quartz solid solution and/or β-quartz solid solution), a stoichiometric lithium alumosilicate (e.g., eucryptite, spodumene, spodumene solid solution, and/or petalite), a non-stoichiometric lithium alumosilicate phase (e.g. Li₂O·Al₂O₃·7.5SiO₂), lithium phosphate, a magnesium silicate (e.g., enstatite), an alkalizirconium silicate (e.g., sogdianite or zektzerite), diopside or wollastonite.

Section B may have a main crystal phase which is the same as the main crystal phase of section C or which is the same as the main crystal phase of section A. Section B may comprise one or more minor crystal phases. The one or more minor crystal phases may be, but is not limited to, lithium disilicate, lithium silicate, apatite (e.g., fluorapatite), one or more quartz crystal phases (e.g., α-quartz, α-quartz solid solution and/or β-quartz solid solution), a stoichiometric lithium alumosilicate (e.g., eucryptite, spodumene, spodumene solid solution, and/or petalite), a non-stoichiometric lithium alumosilicate phase (e.g. Li₂O·Al₂O₃·7.5SiO₂), lithium phosphate, a magnesium silicate (e.g., enstatite), an alkalizirconium silicate (e.g. sogdianite or zektzerite), diopside or wollastonite.

In certain embodiments, section C has a main crystal phase (e.g., lithium disilicate), wherein a content of the respective crystal phase decreases in a direction from section C to section A. This shall be understood in that a content of the crystal phase in section C is higher than a content of the crystal phase in section B, and a content of the crystal phase in section B is higher than a content of the crystal phase in section A. This includes that section A may have a different main crystal phase than section C or that section A has a content of essentially 0 wt.% of a crystal phase which is the main crystal of section C (e.g., when section A is a glass or when section A only contains other crystal phases). It has been found by the inventors that, when a content of the main crystal phase of section C decreases in a direction from section C to section A, it is readily possible to provide a glass-ceramic dental body with specifically advantageous properties, like one or more specific material property gradients as defined herein.

Additionally or alternatively, the content of an amorphous phase (i.e., the glass phase) of the sections may increase in a direction from section C to section A. For example, section C may have a content of an amorphous phase which is lower than the content of an amorphous phase in section B, and section B may have a content of an amorphous phase which is lower than a content of an amorphous phase in section A. Section A may be composed of an amorphous phase (i.e., a glass) or may be composed of a glass-ceramic material. Thus, in one embodiment, the glass-ceramic dental body is characterized in that a content of an amorphous phase (i.e., the glass phase) of the sections increases in a direction from section C to section A.

The one or more crystal phases (e.g., the main crystal phases and the optional one or more minor crystal phases) of section C, section B and section A may be combined in different ways to provide a glass-ceramic dental body with advantageous properties as described herein. With respect to the crystal phases, the glass-ceramic dental body may be categorized into the following three types:
- Type 1: section C and section A with the same main crystal phase
- Type 2: section C and section A with a different main crystal phase
- Type 3: a glass as section A

### 2.1 Type 1: section C and section A with the same main crystal phase

Section C may have the same main crystal phase as section A. In such case, the main crystal phase is typically the same for each one of the sections of the glass-ceramic dental body. In one embodiment, the main crystal phase of each one of the sections is the same (e.g., lithium disilicate, a quartz, or a stoichiometric or non-stoichiometric lithium alumosilicate (LAS)), wherein a content of the main crystal phase decreases from section C to section A. This shall be understood in that a content of the main crystal phase in the bottom phase is higher than a content of the main crystal phase in section B, and a content of the main crystal phase in section B is higher than a content of the main crystal phase in section A.

In one embodiment, the main crystal phase of each one of the sections is the same (e.g., lithium disilicate, a quartz, or a stoichiometric or non-stoichiometric lithium alumosilicate (LAS)), wherein a content of the main crystal phase decreases from section C to section A, and wherein the glass-ceramic dental body is characterized by one or more of:
- a gradient of a biaxial flexural strength, wherein the biaxial flexural strength decreases from section C to section A,
- a gradient of a fracture toughness (K_{IC}), wherein the fracture toughness (K_{IC}) decreases from section C to section A,
- a gradient of a contrast ratio, wherein the contrast ratio decreases from section C to section A,
- a gradient of a coefficient of the thermal expansion, wherein the coefficient of the thermal expansion decreases or increases from section C to section A.

In one embodiment, the main crystal phase of each one of the sections is lithium disilicate, and wherein a content of the lithium disilicate optionally decreases in a direction from section C to section A. In one embodiment, the main crystal phase of each one of the sections is lithium disilicate, and wherein a content of the lithium disilicate optionally decreases in a direction from section C to section A, and wherein section C and section B, and optionally section A, comprises one or more quartz crystal phases e.g., α-quartz and/or α-quartz solid solution), and/or lithium silicate as one or more minor crystal phases. In one embodiment, the main crystal phase of each one of the sections is lithium disilicate, and wherein a content of the lithium disilicate optionally decreases in a direction from section C to section A, and wherein the glass-ceramic dental body is characterized by a gradient of a biaxial flexural strength, wherein the biaxial flexural strength decreases from section C to section A; and/or a gradient of a fracture toughness (K_{IC}), wherein the fracture toughness (K_{IC}) decreases from section C to section A.

In one embodiment, the main crystal phase of each one of the sections is a quartz (e.g., α-quartz, α-quartz solid solution or β-quartz solid solution), and wherein a content of the quartz optionally decreases in a direction from section C to section A. In one embodiment, the main crystal phase of each one of the sections is a quartz (e.g., α-quartz, α-quartz solid solution or β-quartz solid solution), and wherein a content of the quartz optionally decreases in a direction from section C to section A, and wherein section C, section B, and section A, comprises lithium disilicate as a minor crystal phase, and optionally wherein section C and section B comprise a stoichiometric lithium alumosilicate (e.g., spodumene or spodumene solid solution) as another minor crystal phase. In one embodiment, the main crystal phase of each one of the sections is a quartz (e.g., α-quartz, α-quartz solid solution or β-quartz solid solution), and wherein a content of the quartz optionally decreases in a direction from section C to section A, and wherein the glass-ceramic dental body is characterized by a gradient of a contrast ratio, wherein the contrast ratio decreases from section C to section A.

### 2.2 Type 2: section C and section A with a different main crystal phase

It is also possible that section C has a main crystal phase (e.g., lithium disilicate, a quartz, or a stoichiometric or non-stoichiometric lithium alumosilicate (LAS)) which is different to a main crystal phase (e.g., an apatite, a quartz, or a stoichiometric or non-stoichiometric lithium alumosilicate (LAS)) of section A. In such a case, section C and section B may have the same main crystal phase which is different to a main crystal phase of section A. Alternatively, section A and section B may have the same main crystal phase which is different to a main crystal phase of section C. Thus, the main crystal phase may switch from section C to section B or from section B to section A. It is also possible that each one of the sections has a different main crystal phase.

When section C has a main crystal phase which is different to a main crystal phase of section A, the main crystal phase of section C may decrease in its content from section C to section A but is not the main crystal phase of each section. Such a decrease of a crystal phase, which is the main crystal phase of section C, from section C to section A is, for example, shown in connection with Figure 5. Additionally or alternatively, the main crystal phase of section A may decrease in a direction from section A to section C but is not the main crystal phase of each section.

In one embodiment, a main crystal phase (e.g., lithium disilicate, a quartz, or a stoichiometric or non-stoichiometric lithium alumosilicate (LAS)) of section C is different to a main crystal phase (e.g., an apatite, a quartz, or a stoichiometric or non-stoichiometric lithium alumosilicate (LAS)) of section A, and optionally a content of the crystal phase, which is the main crystal phase of section C, decreases in a direction from section C to section A, or a content of the crystal phase, which is the main crystal phase of section A, decreases in a direction from section A to section C. In one embodiment, a main crystal phase (e.g., lithium disilicate, a quartz, or a stoichiometric or non-stoichiometric lithium alumosilicate (LAS)) of section C is different to a main crystal phase (e.g., an apatite, a quartz, or a stoichiometric or non-stoichiometric lithium alumosilicate (LAS)) of section A, and a content of the crystal phase, which is the main crystal phase of section C, decreases in a direction from section C to section A, or a content of the crystal phase, which is the main crystal phase of section A, decreases in a direction from section A to section C. In one embodiment, a main crystal phase (e.g., lithium disilicate, a quartz, or a stoichiometric or non-stoichiometric lithium alumosilicate (LAS)) of section C is different to a main crystal phase (e.g., an apatite, a quartz, or a stoichiometric or non-stoichiometric lithium alumosilicate (LAS)) of section A, and optionally a content of the crystal phase, which is the main crystal phase of section C, decreases in a direction from section C to section A, or a content of the crystal phase, which is the main crystal phase of section A, decreases in a direction from section A to section C, and wherein the glass-ceramic dental body is characterized by one or more of:
- a gradient of a biaxial flexural strength, wherein the biaxial flexural strength decreases from section C to section A,
- a gradient of a fracture toughness (K_{IC}), wherein the fracture toughness (K_{IC}) decreases from section C to section A,
- a gradient of a contrast ratio, wherein the contrast ratio decreases from section C to section A,
- a gradient of a coefficient of the thermal expansion, wherein the coefficient of the thermal expansion decreases or increases from section C to section A.

In one embodiment, the main crystal phase of section C is lithium disilicate and the main crystal phase of section A is an apatite (e.g., fluorapatite) or a quartz (e.g., α-quartz or a α-quartz solid solution or β-quartz solid solution). In another embodiment, the main crystal phase of section C is β-quartz solid solution and the main crystal phase of section A is α-quartz or α-quartz solid solution.

In one embodiment, section C has a main crystal phase which is lithium disilicate, wherein a content of lithium disilicate optionally decreases from section C to section A, and wherein section B and section A have a main crystal phase which is a quartz (e.g., α-quartz, α-quartz solid solution or β-quartz solid solution). In one embodiment, section C has a main crystal phase which is lithium disilicate, wherein a content of lithium disilicate decreases from section C to section A, and wherein section B and section A have a main crystal phase which is a quartz (e.g., α-quartz, α-quartz solid solution or β-quartz solid solution). In one embodiment, section C has a main crystal phase which is lithium disilicate, wherein optionally a content of lithium disilicate decreases from section C to section A, wherein section C comprises one or more quartz crystal phases (e.g., α-quartz α-quartz solid solution and/or β-quartz solid solution) as a minor crystal phase, wherein section B and section A have a main crystal phase which is a quartz (e.g., α-quartz, α-quartz solid solution or β-quartz solid solution), and wherein section B and section A comprise lithium disilicate as a minor crystal phase. In one embodiment, section C has a main crystal phase which is lithium disilicate, wherein a content of lithium disilicate optionally decreases from section C to section A, and wherein section B and section A have a main crystal phase which is a quartz (e.g., α-quartz, α-quartz solid solution or β-quartz solid solution), and wherein the glass-ceramic dental body is characterized by a gradient of a coefficient of the thermal expansion, wherein the coefficient of the thermal expansion decreases or increases from section C to section A, and/or a gradient of a biaxial flexural strength, wherein the biaxial flexural strength decreases from section C to section A; and/or a gradient of a fracture toughness (K_{IC}), wherein the fracture toughness (K_{IC}) decreases from section C to section A.

In one embodiment, section C and section B have a main crystal phase which is lithium disilicate, wherein a content of lithium disilicate optionally decreases from section C to section A, and wherein section A has a main crystal phase which is apatite (e.g., fluorapatite). In one embodiment, section C and section B have a main crystal phase which is lithium disilicate, wherein a content of lithium disilicate optionally decreases from section C to section A, wherein section C and section B comprises one or more quartz crystal phases (e.g., α-quartz and/or one or more quartz solid solutions, like α-quartz solid solution) as a minor crystal phase, and wherein section A has a main crystal phase which is apatite (e.g., fluorapatite). In one embodiment, section C and section B have a main crystal phase which is lithium disilicate, wherein a content of lithium disilicate optionally decreases from section C to section A, and wherein section A has a main crystal phase which is apatite (e.g., fluorapatite), and wherein the glass-ceramic dental body is characterized by a gradient of a biaxial flexural strength, wherein the biaxial flexural strength decreases from section C to section A; and/or a gradient of a fracture toughness (K_{IC}), wherein the fracture toughness (K_{IC}) decreases from section C to section A.

### 2.3 Type 3: a glass as section A

In another embodiment, section A is composed of a glass, and section B and section C are composed of a glass-ceramic material. When section A is composed of a glass, the main crystal phase may be the same or different for section C and section B of the glass-ceramic dental body. The inventors found that, when section A is composed of a glass, it is readily possible to provide a glass-ceramic dental body with specifically advantageous properties, like one or more specific material property gradients as defined herein.

In one embodiment, section A is composed of a glass, and the main crystal phase is the same for section C and section B of the glass-ceramic dental body, wherein a content of the main crystal phase optionally decreases from section C to section B (and to section A taking into consideration that the glass section A is essentially free of crystal phases). In one embodiment, section A is composed of a glass, and section B and section C are composed of a glass-ceramic material, and wherein the glass-ceramic dental body is characterized by one or more of:
- a gradient of a biaxial flexural strength, wherein the biaxial flexural strength decreases from section C to section A,
- a gradient of a fracture toughness (K_{IC}), wherein the fracture toughness (K_{IC}) decreases from section C to section A,
- a gradient of a contrast ratio, wherein the contrast ratio decreases from section C to section A,
- a gradient of a coefficient of the thermal expansion, wherein the coefficient of the thermal expansion decreases or increases from section C to section A.

In one embodiment, section A is composed of a glass, and the main crystal phase of section C and section B is lithium disilicate, wherein a content of the lithium disilicate optionally decreases from section C to section B (and to section A taking into consideration that the glass section A is essentially free of crystal phases). In one embodiment, section A is composed of a glass, and the main crystal phase of section C and section B is lithium disilicate, wherein a content of the lithium disilicate optionally decreases from section C to section A (taking into consideration that the glass section A is essentially free of crystal phases), and wherein the glass-ceramic dental body is characterized by a gradient of a biaxial flexural strength, wherein the biaxial flexural strength decreases from section C to section A; and/or a gradient of a fracture toughness (K_{IC}), wherein the fracture toughness (K_{IC}) decreases from section C to section A, and/or a gradient of a contrast ratio, wherein the contrast ratio decreases from section C to section A.

### 2.4 Further specific combination of crystal phases

The glass-ceramic dental body may be characterized by specific combination of crystal phases according to embodiments as defined in any one of tables I to III herein below.

The glass-ceramic dental body may comprise sections having a main crystal phase according to any one of embodiments AA to EE as defined in table I herein below.

**Table I:**

| **Embodiment** | **Section C** | **Section B** | **Section A** |
|---|---|---|---|
| | Main crystal phase | Main crystal phase | Main crystal phase |
| AA | Lithium disilicate | Lithium disilicate | Lithium disilicate |
| BB | Lithium disilicate | Lithium disilicate | None (glass) |
| CC | Lithium disilicate | Quartz | Quartz |
| DD | Quartz | Quartz | Quartz |
| EE | Lithium disilicate | Lithium disilicate | Apatite |

The glass-ceramic dental body may comprise sections having a main crystal phase according to any one of embodiments A to F as defined in table II herein below.

**Table II:**

| **Embodiment** | **Section C** | **Section B** | **Section A** |
|---|---|---|---|
| | Main crystal phase | Main crystal phase | Main crystal phase |
| A | Lithium disilicate | Lithium disilicate | Lithium disilicate |
| B | Lithium disilicate | Lithium disilicate | none (glass) |
| C | Lithium disilicate | α-quartz or α-quartz solid solution | α-quartz or α-quartz solid solution |
| D | Lithium disilicate | α-quartz solid solution | β-quartz solid solution |
| E | β-quartz solid solution | α-quartz solid solution | α-quartz solid solution |
| F | Lithium disilicate | Lithium disilicate | fluorapatite |

The glass-ceramic dental body may comprise sections having a main crystal phase and comprising one or more (or all) of the minor crystal phases according to any one of embodiments a to j as defined in table III herein below.

**Table III:**

| **Embodiment** | **Section C** | | **Section B** | | **Section A** | |
|---|---|---|---|---|---|---|
| | Main crystal phase | Minor crystal phase | Main crystal phase | Minor crystal phase | Main crystal phase | Minor crystal phase |
| a | Lithium disilicate | α-quartz, quartz solid solution, lithium phosphate | Lithium disilicate | α-quartz, quartz solid solution, lithium phosphate | Lithium disilicate | α-quartz, quartz solid solution, enstatite, lithium phosphate |
| b | Lithium disilicate | α-quartz solid solution, lithium phosphate | Lithium disilicate | α-quartz solid solution, lithium phosphate | Lithium disilicate | lithium phosphate |
| c | Lithium disilicate | α-quartz solid solution, lithium phosphate | Lithium disilicate | α-quartz solid solution, lithium phosphate, apatite | none (glass) | none (glass) |
| d | Lithium disilicate | lithium phosphate | Lithium disilicate | Lithium silicate, lithium phosphate | none (glass) | none (glass) |
| e | Lithium disilicate | α-quartz solid solution, lithium phosphate | α-quartz solid solution | Lithium disilicate, lithium phosphate | α-quartz solid solution | Lithium disilicate, lithium phosphate |
| f | Lithium disilicate | α-quartz solid solution, lithium phosphate | α-quartz solid solution | Lithium disilicate, lithium phosphate | β-quartz solid solution | Lithium disilicate, lithium phosphate |
| g | β-quartz solid solution | Lithium disilicate, spodumene solid solution, lithium phosphate | α-quartz solid solution | Lithium disilicate, spodumen solid solution, lithium phosphate | α-quartz solid solution | Lithium disilicate, lithium phosphate |
| h | Lithium disilicate | α-quartz solid solution, lithium phosphate | Lithium disilicate | α-quartz solid solution, lithium phosphate | fluorapatite | none |
| i | Lithium disilicate | α-quartz, lithium phosphate | α-quartz | Lithium disilicate, lithium phosphate | α-quartz | Lithium disilicate, lithium phosphate |
| j | Lithium disilicate | none | Lithium disilicate | Lithium metasilicate | Lithium disilicate | Lithium metasilicate |

In any one of the embodiments AA to EE of table I, embodiments A to F of table II or embodiments a to j of table III, a content of the crystal phase, which is the main crystal phase in section C, may decrease from section C to section A.

Additionally or alternatively to the gradients and/or the crystal phases, the glass-ceramic dental body may be characterized by its chemical composition. This is described in more detail in the following section.

### 3. Chemical composition

Each one of the sections of the glass-ceramic dental body has a chemical composition that differs from a chemical composition of the other sections. The chemical compositions of each one of the sections typically differ from one another such that each one of the sections comprises at least one (e.g., one, two or three) crystal phase in a content which differs from a content of the respective crystal phase in the other sections.

The chemical composition of each section of the glass-ceramic dental body may be adjusted or selected in such a way that the respective section comprises one or more crystal phases (like a main crystal phase, and optionally one or more minor crystal phases) as defined herein. This can be understood in that, for example, the chemical composition of section C is adjusted or selected in such a way that the one or more crystal phases of section C of the glass-ceramic dental body can be obtained when preparing the glass-ceramic dental body, e.g., by heating (e.g., sintering or hot compacting) a body of glass powders and/or glass-ceramic powders as described herein in connection with the process according to the present invention.

For example, when the chemical composition of a section of the glass-ceramic dental body is to be adjusted or selected in such a way that the section comprises an α-quartz crystal phase of high purity, then the chemical composition needs to be selected to be essentially free of Al₂O₃, Ga₂O₃, and/or In₂O₃. Some residual amount of said components may be unavoidable due to impurities in raw materials. On the other hand, when the chemical composition of a section of the glass-ceramic dental body is to be adjusted or selected in such a way that the section comprises an α-quartz solid solution crystal phase, then the chemical composition needs to be selected to contain Al₂O₃ (or alternatively Ga₂O₃ or In₂O₃). When the chemical composition of a section of the glass-ceramic dental body is to be adjusted or selected in such a way that the section comprises a stoichiometric or non-stoichiometric lithium aluminosilicate crystal phase, then the chemical composition needs to be selected to contain Al₂O₃. Other examples will be apparent to the person of skill in the art.

### 3.1 Section C

Section C may comprise SiO₂, Li₂O, and an alkaline metal oxides other than Li₂O (e.g., K₂O, Na₂O, Rb₂O, and/or CszO). Further, section C typically comprises a nucleating agent (e.g., P₂O₅ and/or a metal, like Cu). Additional components may be, but are not limited to, GeO₂, CaO, MgO, SrO, ZnO, Y₂O₃, Al₂O₃, La₂O₃, ZrO₂, or colouring and/or fluorescent components (e.g., selected from the group of oxides consisting of Sn, Ce, V, Mn, Co, Ni, Cu, Fe, Cr, Tb, Eu, Er, Pr and mixtures thereof).

Section C may comprise (may essentially consist of or may consist of) the following components:
60.0 to 85.0 wt.% of SiO₂,
0.0 to 10.0 wt.% of GeO2,
5.0 to 20.0 wt.% of Li₂O,
0.5 to 15.0 wt.% of alkaline metal oxides other than Li₂O (e.g., K₂O, Na₂O, Rb₂O, and/or Cs₂O),
0.0 to 8.0 wt.% of P₂O₅ (e.g., 0.5 to 8.0 wt.% of P₂O₅),
0.0 to 12.0 wt.% of CaO, MgO, SrO, ZnO or mixtures thereof,
0.0 to 20.0 wt.% of Y₂O₃, Al₂O₃, La₂O₃, or a mixture thereof,
0.0 to 12.0 wt.% of ZrO₂,
0.0 to 5.0 wt.% of colouring and/or fluorescent components, optionally selected from the group of oxides consisting of Sn, Ce, V, Mn, Co, Ni, Cu, Fe, Cr, Tb, Eu, Er, Pr and mixtures thereof, and
0.0 to 1.0 wt.%, optionally 0.0 to 0.5 wt.%, of other components (e.g., F, B₂O₃, metals and/or residual impurities).

Each one of K₂O, Na₂O, Rb₂O, and Cs₂O may be present in section C in an amount of 0.0 to 12.0 wt.%, wherein the amounts are selected such that the K₂O, Na₂O, Rb₂O, and/or Cs₂O make up 0.5 to 15.0 wt.% of section C. The amount of SiO₂ may be adjusted to an amount of GeO₂. For example, when an amount of GeO₂ is present, the amount of SiO₂ can be reduced by that amount.

The chemical composition may differ depending on the main crystal phase (and the one or more minor crystal phases) being present in section C. The chemical composition of section C may be selected in such a way that section C of the glass-ceramic dental body has a main crystal phase which is lithium disilicate or a quartz (e.g., α-quartz, α- or β-quartz solid solution). The chemical composition may be selected in such a way that section C of the glass-ceramic dental body has a main crystal phase, and optionally one or more minor crystal phases, as described herein above for section C.

The components of section C as described herein above may be selected to add up to 100.0 wt.%, based on the total weight of section C.

### 3.2 Section B

Section B may comprise SiO₂, Li₂O, and an alkaline metal oxides other than Li₂O (e.g., K₂O, Na₂O, Rb₂O, and/or Cs₂O). Section B typically comprises a nucleating agent (e.g., CuO, P₂O₅ and/or a metal, like Cu). Additional components may be, but are not limited to, F, B₂O₃, GeO₂, CaO, MgO, SrO, ZnO, Y₂O₃, Al₂O₃, La₂O₃, ZrO₂, or colouring and/or fluorescent components (e.g., selected from the group of oxides consisting of Sn, Ce, V, Mn, Co, Ni, Cu, Fe, Cr, Tb, Eu, Er, Pr and mixtures thereof).

Section B may comprise (may essentially consist of or may consist of) the following components:
55.0 to 80.0 wt.% of SiO₂,
0.0 to 10.0 wt.% of GeO₂,
5.0 to 30.0 wt.% of Li₂O,
0.5 to 15.0 wt.% of alkaline metal oxides other than Li₂O (e.g., K₂O, Na₂O, Rb₂O, and/or Cs₂O),
0.0 to 8.0 wt.% of P₂O₅ (e.g., 0.5 to 8.0 wt.% of P₂O₅),
0.0 to 12.0 wt.% of CaO, MgO, SrO, ZnO or mixtures thereof,
0.0 to 20.0 wt.% of Y₂O₃, Al₂O₃, La₂O₃, or a mixture thereof,
0.0 to 12.0 wt.% of ZrO₂,
0.0 to 5.0 wt.% of colouring and/or fluorescent components, optionally selected from the group of oxides consisting of Sn, Ce, V, Mn, Co, Ni, Cu, Fe, Cr, Tb, Eu, Er, Pr and mixtures thereof, and
0.0 to 1.0 wt.%, optionally 0.0 to 0.5 wt.% of one or more other components (e.g., F, B₂O₃, metals and/or residual impurities).

Each one of K₂O, Na₂O, Rb₂O, and Cs₂O may be present in section B in an amount of 0.0 to 12.0 wt.%, wherein the amounts are selected such that the K₂O, Na₂O, Rb₂O, and/or Cs₂O make up 0.5 to 15.0 wt.% of section B. The amount of SiOz may be adjusted to an amount of GeOz. For example, when an amount of GeOz is present, the amount of SiO₂ can be reduced by that amount.

The chemical composition may differ depending on the main crystal phase (and the one or more minor crystal phases) being present in section B. The chemical composition of section B may be selected in such a way that section B of the glass-ceramic dental body has a main crystal phase which is lithium disilicate or a quartz (e.g., α-quartz, a β-quartz solid solution or α-quartz solid solution). The chemical composition may be selected in such a way that section B of the glass-ceramic dental body has a main crystal phase, and optionally one or more minor crystal phases, as described herein above for section B.

The components of section B as described herein above may be selected to add up to 100.0 wt.%, based on the total weight of section B.

### 3.3 Section A

The chemical composition of section A may differ depending on whether section A is composed of a glass or of a glass-ceramic. When section A is composed of a glass ceramic, the chemical composition may differ depending on the main crystal phase (and the one or more minor crystal phases) being present in section A.

The chemical composition of section A may be selected such that section A has a main crystal phase which is lithium disilicate, a quartz (e.g., α-quartz, a β-quartz solid solution or α-quartz solid solution). In such case, section A may comprise SiO₂, Li₂O, an alkaline metal oxides other than Li₂O (e.g., K₂O, Na₂O, Rb₂O, and/or CszO), and typically a nucleating agent (e.g., , P₂O₅ and/or a metal). Additional components may be, but are not limited to, F, B₂O₃, GeO₂, CaO, MgO, SrO, ZnO, Y₂O₃, Al₂O₃, La₂O₃, ZrO₂, or colouring and/or fluorescent components (e.g., selected from the group of oxides consisting of Sn, Ce, V, Mn, Co, Ni, Cu, Fe, Cr, Tb, Eu, Er, Pr and mixtures thereof).

In one embodiment, section A is composed of a glass-ceramic (e.g., having lithium disilicate or a quartz as a main crystal phase), wherein section A comprises (essentially consists of or consists of) the following components:
59.0 to 90.0 wt.% of SiO₂,
0.0 to 15.0 wt.% of GeO₂,
2.0 to 20.0 wt.% of Li₂O,
0.0 to 15.0 wt.% of alkaline metal oxides other than Li₂O (e.g., K₂O, Na₂O, Rb₂O, and/or Cs₂O),
0.0 to 9.0 wt.% of P₂O₅,
0.0 to 12.0 wt.% of CaO, MgO, SrO, ZnO or mixtures thereof,
0.0 to 20.0 wt.% of Y₂O₃, Al₂O₃, La₂O₃, or a mixture thereof,
0.0 to 12.0 wt.% of ZrO₂,
0.0 to 5.0 wt.% of colouring and/or fluorescent components, optionally selected from the group of oxides consisting of Sn, Ce, V, Mn, Co, Ni, Cu, Fe, Cr, Tb, Eu, Er, Pr and mixtures thereof, and 0.0 to 1.0 wt.%, optionally 0.0 to 0.5 wt.%, of one or more other components (e.g., F, B₂O₃, metals and/or residual impurities).

In one more specific embodiment, section A is composed of a glass-ceramic (e.g., having lithium disilicate or a quartz as a main crystal phase), wherein section A comprises (essentially consists of or consists of) the following components:
60.0 to 80.0 wt.% of SiO₂,
0.0 to 5.0 wt.% of GeO₂,
5.0 to 20.0 wt.% of Li₂O,
0.5 to 15.0 wt.% of alkaline metal oxides other than Li₂O (e.g., K₂O, Na₂O, Rb₂O, and/or Cs₂O),
0.5 to 8.0 wt.% of P₂O₅,
0.0 to 12.0 wt.% of CaO, MgO, SrO, ZnO or mixtures thereof,
0.0 to 20.0 wt.% of Y₂O₃, Al₂O₃, La₂O₃, or a mixture thereof,
0.0 to 12.0 wt.% of ZrO₂,
0.0 to 5.0 wt.% of colouring and/or fluorescent components, optionally selected from the group of oxides consisting of Sn, Ce, V, Mn, Co, Ni, Cu, Fe, Cr, Tb, Eu, Er, Pr and mixtures thereof, and 0.0 to 0.5 wt.% of one or more other components (e.g., F, B₂O₃, metals and/or residual impurities).

Each one of K₂O, Na₂O, Rb₂O, and Cs₂O may be present in section A in an amount of 0.0 to 12.0 wt.%, wherein the amounts are selected such that the K₂O, Na₂O, Rb₂O, and/or Cs₂O make up 0.5 to 15.0 wt.% of section A. The amount of SiOz may be adjusted to an amount of GeOz. For example, when an amount of GeO₂ is present, the amount of SiO₂ can be reduced by that amount.

Alternatively, the chemical composition of section A may be selected in such a way that section A of the glass-ceramic dental body has a main crystal phase which is apatite (e.g., fluorapatite). In such case, section A may comprise SiO₂, K₂O, Na₂O, CaO, and a typically nucleating agent (e.g., , P₂O₅ and/or a metal). Additional components may be, but are not limited to, Li₂O, TiO₂, F, B₂O₃, GeO₂, CaO, MgO, SrO, ZnO, Y₂O₃, Al₂O₃, La₂O₃, ZrO₂, or colouring and/or fluorescent components (e.g., selected from the group of oxides consisting of Sn, Ce, V, Mn, Co, Ni, Cu, Fe, Cr, Tb, Eu, Er, Pr and mixtures thereof).

In one embodiment, section A is composed of a glass-ceramic (e.g., having an apatite as a main crystal phase), wherein section A comprises (essentially consists of or consists of) the following components:
50.0 to 75.0 wt.% of SiO₂,
0.0 to 12.0 wt.% of K₂O,
0.0 to 12.0 wt.% of Na₂O,
0.0 to 12.0 wt.% of CaO,
0.5 to 10.0 wt.% of P₂O₅,
0.0 to 12.0 wt.% of Li₂O,
0.0 to 20.0 wt.% of SrO,
0.0 to 8.0 wt.% of ZnO,
0.0 to 8.0 wt.% of Al₂O₃,
0.0 to 8.0 wt.% of ZrO₂, TiOz or a mixture thereof,
0.0 to 3.0 wt.% of F,
0.0 to 5.0 wt.% of B₂O₃,
0.0 to 5.0 wt.% of colouring and/or fluorescent components, optionally selected from the group of oxides consisting of Sn, Ce, V, Mn, Co, Ni, Cu, Fe, Cr, Tb, Eu, Er, Pr and mixtures thereof,
0.0 to 1.0 wt.%, optionally 0.0 to 0.5 wt.%, of one or more other components (e.g., metals and/or residual impurities).

In one more specific embodiment, section A is composed of a glass-ceramic (e.g., having an apatite as a main crystal phase), wherein section A comprises (essentially consists of or consists of) the following components:
50.0 to 75.0 wt.% of SiO₂,
1.0 to 12.0 wt.% of K₂O,
1.0 to 12.0 wt.% of Na₂O,
1.0 to 12.0 wt.% of CaO,
0.5 to 8.0 wt.% of P₂O₅,
0.0 to 12.0 wt.% of Li₂O,
0.0 to 20.0 wt.% of SrO,
0.0 to 8.0 wt.% of ZnO,
0.0 to 8.0 wt.% of Al₂O₃,
0.0 to 8.0 wt.% of ZrO₂, TiO₂ or a mixture thereof,
0.0 to 3.0 wt.% of F,
0.0 to 5.0 wt.% of B₂O₃,
0.0 to 5.0 wt.% of colouring and/or fluorescent components, optionally selected from the group of oxides consisting of Sn, Ce, V, Mn, Co, Ni, Cu, Fe, Cr, Tb, Eu, Er, Pr and mixtures thereof,
0.0 to 0.5 wt.% of one or more other components (e.g., metals and/or residual impurities).

The chemical composition of section A may be selected in such a way that section A of the glass-ceramic dental body has a main crystal phase, and optionally one or more minor crystal phases, as described herein above for section A.

In yet another alternative, the chemical composition of section A may be selected in such a way that section A is composed of a glass. In such case, section A may comprise SiO₂, K₂O, and Na₂O. Additional components may be, but are not limited to, LizO, F, B₂O₃, CaO, MgO, SrO, ZnO, Al₂O₃, or colouring and/or fluorescent components (e.g., selected from the group of oxides consisting of Sn, Ce, V, Mn, Co, Ni, Cu, Fe, Cr, Tb, Eu, Er, Pr and mixtures thereof).

In one embodiment, section A is composed of a glass, wherein section A comprises (essentially consists of or consists of) the following components:
59.0 to 90.0 wt.% of SiO₂,
0.0 to 20.0 wt.% of E¹(I)₂O,
0.0 to 15.0 wt.% of E²(II)O,
0.0 to 15.0 wt.% of E³(III)₂O₃,
0.0 to 8.0 wt.% of E⁴(IV)O₂,
0.0 to 15.0 wt.% of E⁵(V)₂O₅,
0.0 to 8.0 wt.% of E⁶(VI)O₃,
0.0 to 2.0 wt.% of mixed valence metal oxide,
0.0 to 5.0 wt.% of F,
0.0 to 1.0 wt.% of one or more other components (e.g., further coloring and/or fluorescent components, and/or residual impurities),
wherein
E¹(I)₂O is selected from one valent metal oxides (Me¹(I)₂O) and mixtures thereof,
E²(II) O is selected from two valent metal oxides (Me²(II)O) and mixtures thereof,
E³(III)₂O₃ is selected from three valent metal oxides (Me³(III)₂O₃) and mixtures thereof,
E⁴(IV)O₂ is selected from four valent metal oxides (Me⁴(IV)O₂) and mixtures thereof,
E⁵(V)₂O₅ is selected from five valent metal oxides (Me⁵(V)₂O₅) and mixtures thereof, and
E⁶(VI)O₃ is selected from six valent metal oxides (Me⁶(VI)O₆) and mixtures thereof.

The one valent metal oxides (Me¹(I)₂O) may be selected from Na₂O, Li₂O, K₂O, Rb₂O, Cs₂O and mixtures thereof.

The two valent metal oxides (Me²(II) O) may be selected from CaO, BaO, MgO, SrO, ZnO, SnO and mixtures thereof.

The three valent metal oxides (Me³(III)₂O₃) may be selected from Al₂O₃, La₂O₃, B₂O₃, Y₂O₃, Yb₂O₃, Ga₂O₃, In₂O₃, and mixtures thereof.

The four valent metal oxides (Me⁴(IV)O₂) may be selected from ZrO₂, TiO₂, SnO₂, GeO₂, and mixtures thereof.

The five valent metal oxides (Me⁵(V)₂O₅) may be selected from Ta₂O₅, Nd₂O₅, P₂O₅, and mixtures thereof.

The six valent metal oxides (Me⁶(VI)O₆) may be selected from WO₃, MoOs, and mixtures thereof.

The mixed valence metal oxide may be a mixed three/four valent metal oxide (Me^{3/4}(III,IV)₄O₇).

In one more specific embodiment, section A is composed of a glass, wherein section A comprises (essentially consists of or consists of) the following components:
60.0 to 80.0 wt.% of SiO₂,
1.0 to 18.0 wt.% of K₂O,
0.5 to 12.0 wt.% of Na₂O,
0.0 to 12.0 wt.% of CaO,
0.0 to 12.0 wt.% of Li₂O,
0.0 to 8.0 wt.% of SrO, ZnO or a mixture thereof,
0.0 to 10.0 wt.% of Al₂O₃,
0.0 to 5.0 wt.% of F,
0.0 to 10.0 wt.% of B₂O₃,
0.0 to 15.0 wt. % P₂O₅
0.0 to 5.0 wt.% of colouring and/or fluorescent components, optionally selected from the group of oxides consisting of Sn, Ce, V, Mn, Co, Ni, Cu, Fe, Cr, Tb, Eu, Er, Pr and mixtures thereof, 0.0 to 0.5 wt.% of one or more other components (e.g., residual impurities).

The components of section A as described herein above may be selected to add up to 100.0 wt.%, based on the total weight of section A.

Additionally or alternatively to the gradients, the crystal phases and/or the chemical composition, the glass-ceramic dental body may be characterized by its structure and/or form.

### 4. Structure and form

The glass-ceramic dental body comprises three consecutive sections:
a section A,
a section B, and
a section C,
wherein each one of the sections has a chemical composition that differs from a chemical composition of the other sections.

In one embodiment, the glass-ceramic dental body is composed of the three consecutive sections: a section A, a section B, and a section C.

### 4.1 Dental blank

The glass-ceramic dental body may be a glass-ceramic dental blank, like a dental mill blank or a press blank. The dental blank (e.g., the dental mill blank) may be characterized in that:
section A forms a top section of the dental blank,
section B forms an intermediate section of the dental blank, and
section C forms a bottom section of the dental blank.

The glass-ceramic dental blank is not particularly limited as regards its shape or its dimensions as long as it is suitable for use for preparing a dental restoration (e.g., using a CAD/CAM process or using hot pressing into a mold). The glass-ceramic dental blank may have, but is not limited to, the form of a rectangular block, an ingot, a disc, a cylinder, a dental preform (e.g., an abutment preform or a tooth sector), a cone, a cone segment, a pyramid, or a pyramid segment. The glass-ceramic dental blank may comprise additional components on the outsides of a glass-ceramic dental blank which are not part of the sections as described herein. For example, the dental blank may comprise on its outsides a holding pin, a support layer, a protective layer, a printing layer, or a sacrificial layer, like a (thin) layer of a glass-ceramic material which is removed (e.g., by milling) when forming a dental restoration from the blank. In one embodiment, the dental blank is a dental mill blank having the form of a disc, a cylinder or a rectangular block. The dental mill blank may comprise a holding pin for holding the glass-ceramic dental mill blank in a device when machining it, e.g., using a CAD/CAM process. A holding pin is not to be considered a part of a section of the dental mill blank.

Each section of the glass-ceramic dental blank may have a specific height relative to the overall height of the glass-ceramic dental blank. The overall height of the glass-ceramic dental blank can be understood as the dimension of the glass-ceramic dental blank in a z-direction. The z-direction is the direction which intersects each one of the section. For example, for rectangular or disc-shaped dental blanks, and the like, the overall height can be determined as a distance of a perpendicular line between an outer surface of top section to an opposing outer surface of the bottom, the perpendicular line intersecting all sections of the dental blank. The height of a section is to be understood as a maximum height of a section in a z-direction. This is irrespective of whether a relative height or an absolute height is defined herein. Thus, the definition of a height of a section (e.g., a relative height or an absolute height) as used herein does not necessarily mean that the height of the section is constant, although this is possible.

Each one of the sections may have a substantially constant height. A "substantially constant height" means that the height of a section does not vary by more than 5%, relative to an average height of the section. The bottom section may have a height in the range of 30 to 75%, 40 to 75 %, 45 to 70% or 50 to 70%, relative to the overall height of the glass-ceramic dental blank. The intermediate section may have a height in the range of 5 to 40%, 10 to 30%, or 15 to 25%, relative to the overall height of the glass-ceramic dental blank. The top section may have a height in the range of 5 to 35%, 10 to 30%, or 15 to 25 %, relative to the overall height of the glass-ceramic dental blank.

### 4.2 Dental restoration

The glass-ceramic dental body may be a dental restoration. The dental restoration may be characterized in that:
section A forms an upper part of the dental restoration (e.g., a part of or all of an incisal zone),
section B forms an intermediate part of the dental restoration (e.g., a part of or all of a transitional zone between an incisal zone and a dentin zone), and
section C forms a lower part the dental restoration (e.g., a part of or all of a dentin zone).

The dental restoration may be, but is not limited to, a crown, a partial crown, an abutment, an abutment crown, an inlay, an onlay, a veneer, a shell, or a multi-unit framework, or a bridge (e.g., 2-, 3- or 4- unit bridge) an implant bridge, and the like. The dental restoration may have a desirable color. The dental restoration may have a color that matches a shade of by the VITA classical A1-D4^{®} shade guide with VITA Bleached Shades manufactured by Vita Zahnfabrik. The shade may be, but is not limited to, A1, A2, A3.5, A4, B1, B2, B3, B4, C1, C2, C3, C4, D1, D2, D3, D4, BL1 or BL2.

The dental restoration may comprise a glazing. In one embodiment, the dental restoration comprises at least a first glazing and a second glazing, wherein the first glazing has a coefficient of thermal expansion which is lower than the coefficient of thermal expansion of the second glazing, and wherein the first glazing is present on a surface area of a part of the dental restoration having a lower coefficient of thermal expansion, and the second glazing is present on a surface area of a part of the dental restoration having a higher coefficient of thermal expansion. In that context, the terms "lower" and "higher" are to be understood in relation to one another.

### 4.3 Structure

The glass-ceramic dental body may have a multi-layer structure. Each one of the sections A to C may be composed of one or more layers, like 1 to 8 layers, 1 to 6 layers, 1 to 4 layers, 1 to 3 layers, like two layers or one layer. Each one of the layers may differ in their chemical composition. When the glass-ceramic dental body has a multi-layer structure, section A may be a layer A, section C may be a layer C, and section B may be formed by at least one intermediate layer between layer A and layer C, and each layer has a chemical composition that differs from a chemical composition of the other layers. The at least one intermediate layer may be 1 to 8 layers, 1 to 6 layers, 1 to 4 layers, 1 to 3 layers, like two layers or one layer.

When the glass-ceramic dental body has a multi-layer structure, the one or more gradients as described herein may be characterized in that the respective properties of the one or more gradients change in a stepwise manner layer-by-layer from layer C to layer A. Additionally or alternatively, the content of a crystal phase (e.g., a main crystal phase of the bottom layer) may change (e.g., decrease) in a stepwise manner layer-by-layer from layer C to the layer A.

The layers of the glass-ceramic dental body are not particularly limited in terms of their dimensions and shapes. One or more of the layers may be non-planar. For example, one or more layers may have one or two faces (e.g., an interface between two layers or an outside surface depending on the location of the layer in the dental body) that are curved, e.g., that have a positive or negative curvature (e.g., being convex or concave). It is also possible that one or more of the layers have a height that increases uniformly or non-uniformly over at least a part of the layer (e.g., in a conical form). One or more of the layers, optionally all of the layers, may be substantially planar. In that context, "substantially planar" means that a layer is planar, subject to a tolerance of 5% of an average thickness of the layer. The layers of the glass-ceramic dental body may be arranged such that the boundaries of the layers are substantially parallel to each other.

Alternatively, the glass-ceramic dental body has an intermediate section having a chemical composition that gradually changes in a direction from section C to section A. In this alternative, the glass-ceramic dental body typically does not contain discrete layers. Section A and section C may have an essentially homogeneous chemical composition. This can be understood in that section A and section C are parts of the glass-ceramic dental body in which the chemical composition does not change in a direction from section C to section A.

The glass-ceramic dental body is typically a heat-treated (e.g., a (fully) sintered or hot compacted) glass-ceramic dental body. The glass-ceramic dental body may be obtained by heat treating at a maximum temperature in a range of 650 to 1050°C. The glass-ceramic dental body may be obtained by (fully) sintering at a maximum sintering temperature in a range of 700 to 1050 °C, like in a range of 780 to 980°C, like in a range of 820 to 940°C. The glass-ceramic dental body may be obtained by hot compacting at a maximum temperature in a range of 650 to 850 °C, like in a range of 700 to 800°C, and at a pressure in a range of 5 to 50 MPa, like in a range of 10 to 30 MPa.

### II. Process for preparing the glass-ceramic dental body

One aspect of the present invention provides a process for preparing a glass-ceramic dental body according to an embodiment of the present invention. The process comprises the steps of:
- providing two or more powders selected from glass powders, glass-ceramic powders and mixtures thereof;
- preparing a shaped body from the powders; and

- subjecting the shaped body to a heat treatment to obtain a glass-ceramic dental body.

The shaped body typically comprises three consecutive powder sections:
a powder section A,
a powder section B, and
a powder section C,
wherein a weight ratio of the two or more powders differs in each one of the powder sections.

### 1. Providing the glass powders and/or glass-ceramic powders

The process comprises the step of providing two or more powders selected from glass-powders, glass-ceramic powders and mixtures thereof. The powders differ in their chemical composition. In one embodiment, the two or more powders are two or more glass powders.

The two or more powders may be two powders (e.g., two glass powders). It is however also possible to use more than two powders such as three powders (e.g., three glass powders) or more than three powders. In certain embodiments, two powders (i.e., a first powder and a second powder) are used for preparing the glass-ceramic dental body. In certain other embodiments, three powders (i.e., a first powder, a second powder, and a third powder) are used for preparing the glass-ceramic dental body.

Each one of the glass powders is typically provided by melting chemical starting materials which are suitable for preparing a glass. The starting materials may be melted at a temperature between 1300 to 1650°C (e.g., between 1450 to 1650°C) over a time period of 30 min to 10 h (e.g., between 30 min to 3 h). The melt may be poured into water for freezing a glass. To increase homogeneity of the glass, the glass may be re-melted under the same or similar conditions and re-frozen in water. Each one of the obtained glasses may be dried in an oven. Each one of the obtained glasses may be ground to obtain a glass powder. The volume-based median particle size d₅₀ of each one of the glass powders may be in a range of 5 to 30 µm, like in a range of 10 to 20 µm. The volume-based top cut particle size d₉₈ of each one of the glass powders may be below 60 µm, like below 50 µm. The volume-based median particle size d₅₀ and the volume-based top cut particle size d₉₈ may be determined by laser diffraction, e.g., according to ISO 13320 (ISO 13320:2009).

Each one of the powders may be combined with one or more additives such as, but not limited to, one or more pigments, one or more fluorescent pigments, one or more pressing aids and/or one or more binders. Suitable pigments may be, but are not limited to, doped spinels, doped zirconium oxides, zirconium oxide, doped zirconium silicates, doped yittrium silicates or tin oxide. Suitable pressing aids may be, but are not limited, to polyethylene glycols or stearates. Suitable binders may be, but are not limited to, polyvinyl alcohols and cellulose derivatives, such as sodium carboxymethylcellulose.

The powders, optionally in combination with the one or more additives, may be provided in dry form. The powders together with the one or more additives may be provided in form of a dry blend. The dry blend may be a particulate mixture or may be in the form of granules. Granules may be prepared by granulating the powders together with the one or more additives, typically comprising one or more binders, in a granulation process as known in the art. The dry blend may comprise a powder in an amount of at least 90 wt.%, based on the total weight of the dry blend. The one or more additives may be present in the dry blend in an amount in a range of 0.1 to 10.0 wt.%, like 0.3 to 5.0 wt.%, based on the total weight of the dry blend. The one or more binders may be present in the dry blend in an amount in a range of 0.1 to 5.0 wt.%, like 0.3 to 3.0 wt.%, based on the total weight of the dry blend. The one or more pressing aids may be present in the dry blend in an amount in a range of 0.1 to 3.0 wt.%, like 0.1 to 1.0 wt.%, based on the total weight of the dry blend.

Alternatively, the powders, optionally in combination with the one or more additives, may be provided in liquid form, like in a form of a suspension, and typically in the form of an aqueous suspension. The suspension may be a slurry. The suspension may comprise a powder in an amount in a range of 30 to 90 wt.%, like 40 to 70%, based on the total weight of the suspension. The suspension may comprise one or more additives (e.g., one or more pigments, one or more fluorescent pigments, one or more pressing aids and/or one or more binders) in an amount in a range of 0.1 to 10.0 wt.%, like 0.3 to 5.0 wt.%, based on the total dry weight of the suspension. Suitable pigments, fluorescent pigments, pressing aids and binders are known to the skilled person, and may be, but are not limited, to those described above. The one or more binders may be present in an amount in a range of 0.1 to 5.0 wt.%, like 0.3 to 3.0 wt.%, based on the total weight of the dry weight of the suspension. The one or more pressing aids may be present in an amount in a range of 0.1 to 3.0 wt.%, like 0.1 to 1.0 wt.%, based on the total dry weight of the suspension. Additionally or alternatively, the suspension may comprise one or more auxiliary agents, like one or more rheology modifiers (e.g., xanthans or starches), one or more dispersants (e.g., polymers or lecithins), one or more buffers and/or pH adjusting agents (e.g., acids, like acetic acid or hydrochloric acid), and the like. The suspension may comprise the one or more auxiliary agents in an amount in a range of 0.1 to 3.0 wt.%, based on the total weight of the suspension.

### 2. Preparing the shaped body

The process further comprises the step of preparing a shaped body from the powders (e.g., the glass powders). The shaped body may be porous.

The shaped body may be prepared by additive manufacturing such as stereo lithography, inkjet printing, screen printing, powder bed printing or fused deposition modeling (fused filament fabrication). When the shaped body is prepared by additive manufacturing, it may have a shape of a dental restoration so that the shaped body can be converted to a dental restoration in a subsequent step by heat treating (e.g., sintering as described below). Alternatively, the shaped body may have a shape of dental blank so that the shaped body can be converted to a dental blank by heat treating (e.g., sintering as described below).

Alternatively, the shaped body may be prepared by molding the powders in a suitable mold to obtain a molded body. The molded body typically has a pre-shape of a dental blank. The pre-shape may have the desired form of the dental blank such as, but not limited to, a disc, a cylinder or a rectangular block. The preparation of the molded body may differ depending on the form in which the powders are provided. When the powders are provided in dry form, e.g., in form of a dry blend with one or more additives, the powders may be introduced into the mold followed by a compacting step (e.g., a pressing step, like an uniaxial pressing). The compacting step may be a cold compacting step. The cold compacting (e.g., cold pressing) may be carried out at a temperature of less than 60°C, like in a range of 15 to 35°C, and at a pressure of 2 to 30 bar, like in a range of 5 to 15 bar. Additionally or alternatively, the compacting step may be a hot compacting step. The hot compacting step (e.g., hot pressing step) may be carried out at a temperature in a range of 650 to 850°C, like in the range of 700 to 800°C, and a pressure in a range of 5 to 50 MPa, preferably 10 to 30 MPa. The hot compacting is typically carried out over a time period in a range of 0.1 to 10 min, like in a range of 0.3 to 5 min. The hot compacting step is preferably carried at an atmospheric pressure below ambient pressure, for example, at an atmospheric pressure of less than 0.1 bar, like in a range of 0.01 to 0.8 bar. The hot compacting may be a pressure sintering. After the hot compacting, a compacted body may be obtained which is essentially non-porous or which does not contain a significant porosity.

When the powders are provided in liquid form, the powders may be introduced into the mold followed by removing the liquid. The mold may be a mold suitable for die casting or slip casting. The mold may have pores through which the liquid can be removed. Removal of the liquid may be carried out and/or supported by, e.g., pressing, suction, and/or freeze drying.

The shaped body typically comprises (or may be composed of) three consecutive powder sections:
a powder section A,
a powder section B, and
a powder section C.

The powder sections are prepared such that a weight ratio of the two or more powders (e.g., two or more glass powders) differs in each one of the powder sections.

When the glass-ceramic dental body has a multi-layer structure, the powder sections A to C may be composed of one or more powder layers. For example, the shaped body may be prepared such that the powder section C is a powder layer C, the powder section B is formed by at least one intermediate powder layer, and the powder section A is a powder layer A. The powder section B may be formed by 1 to 8 intermediate powder layers or more (e.g., 1 to 6 layers, 1 to 4 layers, 1 to 3 layers, 2 layers, 1 layer).

Alternatively, the molded body may be prepared from the powders in a way that the powder section C and the powder section A contain an essentially homogeneous chemical composition, and the powder section B contains the two or more powders in a weight ratio which gradually changes within powder section B in a direction from the powder section C to the powder section A. Such a gradual change can be achieved by using a deposition device or dosing device known in the art. Such a deposition device or a dosing device can be adjusted to continuously change a weight content of components in a mixture that is added into a mold.

### 2.1 Two powders

When the shaped body (e.g., the molded body) is prepared from two powders (i.e., a first powder and a second powder), the powder sections are prepared such that a weight ratio of the two powders differs in each one of the powder sections. In one embodiment, the shaped body (e.g., the molded body) is prepared from two glass powders (i.e., a first glass powder and a second glass powder), and the powder sections are prepared such that a weight ratio of the two glass powders differs in each one of the powder sections.

The powder sections are typically prepared such that a weight ratio of the first powder to the second powder decreases in a direction from the powder section C to the powder section A. This is to be understood in that a weight content of the first powder decreases in a direction from the powder section C to the powder section A, and a weight content of the second powder increases in a direction from the powder section C to the powder section A. Thus, the powder section C may comprise an amount of the first powder that is higher than an amount of the first powder in powder section B, and powder section B comprises an amount of the first powder that is higher than an amount of the first powder in powder section A (and the other way round for the second glass powder). This includes that the second powder may essentially be absent (i.e., about 0 wt.%) from powder section C and/or the first powder may essentially be absent (i.e., about 0 wt.%) from the powder section A.

The weight ratio of the first powder to the second glass powder may change from a weight ratio of >50:<50, such as >90:<10 (e.g., 100:0), in powder section C to a weight ratio of <50:>50, such as <10:>90 (e.g., 0:100), in powder section A. Thus, a main powder of a powder section (i.e., the powder being present in a powder section in an amount of >50 wt.%, based on the combined weight of the powders) may be different between the powder section C and the powder section A. It is however also possible that the main powder is the same in each one of the powder sections. In that case, the weight ratio of the first powder to the second powder may change from a weight ratio of >90:<10 (e.g., 100:0) in the powder section C to a weight ratio in a range of >50:<50 to <90:>10 (e.g., 60:40) in the powder section A. The weight ratio of the first powder to the second powder in the powder section B lies between the weight ratio in powder section C and the weight ratio in powder section A.

For example, the shaped body (e.g., the molded body) may comprise (or may be composed of)
powder section A, wherein powder section A is composed of the second powder (e.g., the second glass powder), optionally in combination with the one or more additives,
powder section B, wherein powder section B is composed of a mixture of the first powder (e.g., the first glass powder) and the second powder (e.g., the second glass powder), each one optionally in combination with the one or more additives, and
powder section C, wherein powder section C is composed of the first powder (e.g., the first glass powder), optionally in combination with the one or more additives.

When the glass-ceramic dental body has a multi-layer structure, the layers are typically prepared such that a weight ratio of the first powder to the second powder decreases in a direction from a powder layer C to a powder layer A. This is to be understood in that a weight content of the first powder decreases in a direction from the powder layer C to the powder layer A, and a weight content of the second powder increases in a direction from the powder layer C to the powder layer A. The weight content of the first powder decreases layer-by-layer and the weight content of the second powder increases layer-by-layer. Typically, the chemical composition of each layer is essentially homogeneous. Thus, if the molded body comprises 1 to 8 intermediate layers (e.g., 1 to 6 layers, 1 to 4 layers, 1 to 3 layers, 2 layers, 1 layer), the weight content of the first powder and the second powder, respectively, decreases and increases in a stepwise manner layer-by-layer in a direction from powder section C to powder section A. When a layer comprises or is composed of a mixture of the first powder and the second powder (optionally in combination with one or more additives), the powders may be premixed with one another before being introduced into the mold to form the layer.

In an alternative embodiment, the molded body is prepared from the two powders in a way that powder section C and powder section A contain an essentially homogeneous chemical composition, and a weight ratio of the first powder to the second powder gradually decreases within section B in a direction from powder section C to powder section A. The weight content of the second powder gradually decreases within section B in a direction from powder section C to powder section A.

### 2.2 Three powders

When the molded body is prepared from three powders (i.e., a first powder, a second powder and a third powder), the powder sections may be prepared such that a weight ratio of the three powders differs in each one of the powder sections. In one embodiment, the molded body is prepared from three glass powders (i.e., a first glass powder, a second glass powder and a third glass powder), and the powder sections may be prepared such that a weight ratio of the three glass powders differs in each one of the powder sections.

It is possible that each powder section comprises one of the three powders (e.g., one of the three glass powders). Thus, a weight ratio of the first powder to the second powder to the third powder may be 100:0:0 in powder section C, 0:100:0 in powder section B, and 0:0:100 in powder section A.

For example, the shaped body (e.g., the molded body) may comprise (or may be composed of)
powder section A, wherein powder section A is composed of the third powder (e.g., third glass powder), optionally in combination with the one or more additives,
powder section B, wherein powder section B is composed of the second powder (e.g., second glass powder), optionally in combination with the one or more additives, and
powder section C, wherein powder section C is composed of the first powder (e.g., first glass powder), optionally in combination with the one or more additives.

It is however also possible, and sometimes preferred, that one or more of the powder sections (e.g., at least section B) comprises a mixture of two or more of the three powders. This is typically the case when the glass-ceramic dental body has a multi-layer structure having at least 4 layers, or when section B has a gradually changing weight ratio of the powders.

When the glass-ceramic dental body has a multi-layer structure, the powder layers may be prepared such that a weight ratio of the three powders differs in each one of the powder layers.

Alternatively, the molded body may be prepared from the powders in a way that powder section C and powder section A contain an essentially homogeneous chemical composition, and powder section B contains a weight ratio of powders selected from the first powder, the second powder and the third powder which gradually changes within section B in a direction from powder section C to powder section A.

### 3. Heat-treating the shaped body

The process further comprises the step of subjecting the shaped body (e.g., the molded body) to a heat treatment to obtain a glass-ceramic dental body.

The heat treatment typically comprises a sintering step. During the sintering step, the shaped powder body (e.g., molded powder body) is densified by applying heat. The sintering step may be a conventional sintering step which is carried out without applying additional pressure. It is however also possible that the sintering step is a pressure sintering. In pressure sintering, the densification of the powder body is assisted by applying pressure. The pressure sintering may be or may be carried simultaneously with the hot compacting step as described above. The heat treatment may further comprise a crystallization step. In a crystallization step, one or more new crystal phases may be created or one or more existing crystal phases may be converted to another crystal phase. The crystallization step may be carried out together with the sintering step or separately, like after the sintering step. When the shaped body comprises or is composed of glass powder, the heat treatment typically further comprises a crystallization step.

The heat treatment may be carried out at a maximum temperature in a range of 700 to 1050 °C, like in a range of 780 to 980°C, like in a range of 820 to 940°C. The maximum temperature may be hold for a time period in a range of 5 min to 2 hours, like in a range of 10 min to 1 hour, like in a range of 15 min to 45 minutes.

The heat treatment may comprise one or more heating steps, like two, three or more heating steps. For example, the heat treatment may comprise a first heating and a second heating step. The first heating step may start at a temperature in a range of 300 to 500°C, like in a range of 350 to 450°C, and may end at a temperature in a range of 500 to 700°C, like in a range of 550 to 650°C. An end temperature of the first heating step may be hold for a time period in a range of 5 min to 2 hours, like in a range of 10 min to 1 hours, like in a range of 15 min to 45 minutes. The first heating step may have a heating rate in a range of 5 to 15 K/min. The second heating step may start at the end temperature of the first heating step and may end at the maximum temperature. The second heating step may have a heating rate in a range of 5 to 15 K/min.

The heat treatment may partially or fully be carried out at a pressure below ambient pressure. For the present disclosure, ambient pressure is defined as 1.013 bar. For example, the heat treatment may partially or fully be carried out at a pressure of less than 0.9 bar, like in a range of 10 to 300 mbar or 30 to 120 mbar. The heat treatment is typically followed by a cooling step. The cooling step starts at the maximum temperature and ends at a temperature which is suitable for handling the glass-ceramic dental body (like removing the glass ceramic dental body from a sintering furnace).

The process may comprise one or more additional process steps which are typical in the art, and which may be carried out before, between or after the process steps described herein. Additional steps may be, but are not limited to, adjusting the particle size distribution of the powders (e.g., by sieving) and/or preparing the surface of the shaped body (e.g., the molded body) and/or the glass-ceramic dental body (e.g., by grinding, lapping or polishing).

### III. Use of the glass-ceramic body

When the glass-ceramic dental body is a glass-ceramic dental blank (e.g., a dental mill blank), the glass-ceramic dental body is useful for preparing a dental restoration, like a dental restoration as described herein. One embodiment of the present invention relates to the use of the glass-ceramic dental blank according to an embodiment of the present invention for preparing a dental restoration. One embodiment of the present invention relates to a process for preparing a dental restoration, the process comprising using a glass-ceramic dental blank according to an embodiment of the present invention.

In one preferred embodiment, a process is provided for preparing a dental restoration, wherein the process comprises the steps of machining a glass-ceramic dental mill blank according to an embodiment of the present invention to provide a dental restoration, and optionally surface-treating the dental restoration.

The machining of the dental mill blank may be carried out by any conventional process for machining a glass-ceramic dental mill blank, and typically by a CAD/CAM process. Such processes are known in the art. The machining may include, but is not limited to, cutting, drilling and/or grinding the glass-ceramic dental mill blank. The dental restoration may optionally be subjected to a surface-treating as is known in the art, e.g., by polishing, coloring and/or glazing the surface of the dental restoration. In one embodiment, the dental restoration is surface-treated by at least a first glazing and a second glazing, wherein the first glazing has a coefficient of thermal expansion which is lower than the coefficient of thermal expansion of the second glazing, and wherein the first glazing is applied on a surface area of a part of the dental restoration having a lower coefficient of thermal expansion, and the second glazing is applied on a surface area of a part of the dental restoration having a higher coefficient of thermal expansion. In that context, the terms "lower" and "higher" are to be understood in relation to one another.

Another embodiment of the present invention provides a dental restoration which is obtainable using a glass-ceramic dental mill blank according to an embodiment of the present invention. Another embodiment of the present invention provides a dental restoration which is obtainable by a process for preparing a dental restoration according to an embodiment of the present invention.

### IV. Further non-limiting aspects and embodiments

Further non-limiting aspects and embodiments of the present invention are defined in the following items [1] to [35]:
[1] A glass-ceramic dental body comprising (or being composed of) three consecutive sections:
   a section A,
   a section B, and
   a section C,
   wherein each one of the sections has a chemical composition that differs from a chemical composition of the other sections.
[2] The glass-ceramic dental body according to item [1], wherein the glass ceramic-dental body is characterized by one or more gradients of a mechanical property in a direction from section C to section A.
[3] The glass-ceramic dental body according to item [2], wherein the one or more gradient of a mechanical property is a gradient of a biaxial flexural strength, wherein the biaxial flexural strength decreases from section C to section A; and/or a gradient of a fracture toughness (K_{IC}), wherein the fracture toughness (K_{IC}) decreases from section C to section A.
[4] The glass-ceramic dental body according to any one of items [1] to [3], wherein the glass ceramic-dental body is characterized by a gradient of an optical property in a direction from section C to section A.
[5] The glass-ceramic dental body according to item [4], wherein the gradient of an optical property is a gradient of a contrast ratio, wherein the contrast ratio decreases from section C to section A.
[6] The glass-ceramic dental body according to any one of items [1] to [5], wherein the glass ceramic-dental body is characterized by a gradient of a thermal property in a direction from section C to section A.
[7] The glass-ceramic dental body according to item [6], wherein the gradient of a thermal property is a gradient of a coefficient of the thermal expansion, wherein the coefficient of the thermal expansion decreases or increases from section C to section A.
[8] The glass-ceramic dental body according to item [7], wherein the coefficient of the thermal expansion decreases from section C to section A.
[9] The glass-ceramic dental body according to item [7], wherein the coefficient of the thermal expansion increases from section C to section A.
[10] The glass-ceramic dental body according to any one of items [1] to [9], wherein a main crystal phase of each one of section C, section B and section A is the same.
[11] The glass-ceramic dental body according to item [10], wherein a content of the main crystal phase decreases in a direction from section C to section A.
[12] The glass-ceramic dental body according to any one of items [1] to [9], wherein a main crystal phase of section C is different to a main crystal phase of section A.
[13] The glass-ceramic dental body according to item [12], wherein a content of the crystal phase, which is the main crystal phase of section C, decreases in a direction from section C to section A.
[14] The glass-ceramic dental body according to item [12], wherein a content of the crystal phase, which is the main crystal phase of section A, decreases in a direction from section A to section C.
[15] The glass-ceramic dental body according to any one of items [1] to [9], wherein section A is composed of a glass, and wherein section B and section C are composed of a glass-ceramic.
[16] The glass-ceramic dental body according to any one of items [1] to [15], wherein section C has a main crystal phase, and a content of this crystal phase decreases from section C to section B.
[17] The glass-ceramic dental body according to any one of items [1] to [16], wherein a main crystal phase of section C is lithium disilicate, a quartz, or a stoichiometric or non-stoichiometric lithium alumosilicate (LAS).
[18] The glass-ceramic dental body according to item [17], wherein the main crystal phase of section C is lithium disilicate.
[19] The glass-ceramic dental body according to item [17], wherein the main crystal phase of section C is a quartz (e.g., α-quartz, α-quartz solid solution, or β-quartz solid solution).
[20] The glass-ceramic dental body according to any one of items [1] to [19], wherein a main crystal phase of section C is not lithium metasilicate.
[21] The glass-ceramic dental body according to any one of items [1] to [9], [12] to [14], and [16] to [20], wherein a main crystal phase of section A is an apatite, a quartz, or a stoichiometric or non-stoichiometric lithium alumosilicate (LAS).
[22] The glass-ceramic dental body according to item [21], wherein the main crystal phase of section A is a quartz (e.g., α-quartz, α-quartz solid solution, or β-quartz solid solution).
[23] The glass-ceramic dental body according to item [21], wherein the main crystal phase of section A is an apatite (e.g., fluoroapatite).
[24] The glass-ceramic dental body according to any one of items [1] to [23], wherein section A comprises one or more crystal phases which are different to a crystal phase in section C.
[25] The glass-ceramic dental body according to any one of items [1] to [24], wherein the glass-ceramic dental body is a dental blank, like a dental mill blank or a dental press blank.
[26] The glass-ceramic dental body according to item [25], wherein:
   section A forms a top section of the dental blank,
   section B forms an intermediate section of the dental blank, and
   section C forms a bottom section of the dental blank.
[27] The glass-ceramic dental body according to any one of items [1] to [24], wherein the glass-ceramic dental body is a dental restoration.
[28] The glass-ceramic dental body according to item [27], wherein:
   section A forms an upper part of the dental restoration, like a part of or all of an incisal zone,
   section B forms an intermediate part of the dental restoration, like a part of or all of a transitional zone between an incisal zone and a dentin zone, and
   section C forms a lower part the dental restoration, like a part of or all of a dentin zone.
[29] The glass-ceramic dental body according to any one of items [1] to [28], having a multi-layer structure, wherein section A is a layer A, section C is a layer C, and section B is formed by at least one intermediate layer between layer A and C, and each one of the layers has a chemical composition that differs from a chemical composition of the other layers.
[30] The glass-ceramic dental body according to any one of items [1] to [28], wherein section A and section C have a homogenous chemical composition, and section B has a chemical composition that gradually changes in a direction from section C to section A.
[31] A process for preparing a glass-ceramic dental body according to any one of items [1] to [30], the process comprising the steps of:
   - providing two or more powders selected from glass powders, glass-ceramic powders and mixtures thereof;
   - preparing a shaped body from the powders, and
   - subjecting the shaped body to a heat treatment to obtain a glass-ceramic dental body.
[32] The process according to item [31], wherein the shaped body comprises (or is composed of) three consecutive powder sections:
   a powder section A,
   a powder section B, and
   a powder section C,
   wherein a weight ratio of the two or more powders differs in each one of the powder sections.
[33] Use of a glass-ceramic dental body according to any one of items [25], [26], [28] and [29] for preparing a dental restoration.
[34] A process for preparing a dental restoration, wherein the process comprises the steps of machining a glass-ceramic dental body according to any one of items [25], [26], [28] and [29] to provide a dental restoration, and optionally surface-treating the dental restoration.
[35] A dental restoration that is obtainable by using a glass-ceramic dental body according to any one of items [25], [26], [28] and [29] or by the process according to item [34].

In the following, the present invention is described by way of specific examples which shall not be construed as limiting the invention in any way.

### V. Example section

### 1. Measuring methods

### 1.1 Biaxial flexural strength

The biaxial flexural strength was determined according to DIN EN ISO 6872 (DIN EN ISO 6872:2019). The test bodies were obtained from the different sections of the glass-ceramic dental body by cutting the respective section of the glass-ceramic dental body with diamond-coated tools.

### 1.2 Fracture toughness K_{IC}

The fracture toughness (K_{IC}) was determined according to the Single Edge V-Notched Beam (SEVNB) method according to DIN EN ISO 6872, and particularly DIN EN ISO 6872:2015. The test bodies were obtained from the different sections of the glass-ceramic dental body by cutting the respective section of the glass-ceramic dental body with diamond-coated tools.

### 1.3 Contrast ratio

The contrast ratio was determined according to BS 5612, and particularly BS 5612:1978. The contrast ratio was determined using test bodies having a thickness of 2 mm ± 0.02 mm. The test bodies were obtained from the different sections of the glass-ceramic dental body by cutting the respective section of the glass-ceramic dental body with diamond-coated tools. The measurements were made using a spectrophotometer CM 3700-D (Konica-Minolta). Prior to the measurement, the cut section were wet ground to a thickness of 2 mm ± 0.02 mm using a rotating diamond grinding disc and 1000 SiC grinding paper, followed by a final surface wet-polishing using a diamond grind disc (20 µm).

### 1.4 Coefficient of thermal expansion (CTE)

The coefficient of thermal expansion (CTE) was determined using a dilatometer according to DIN EN ISO 6872, and particularly DIN EN ISO 6872:2015. The test bodies were obtained from the different sections of the glass-ceramic dental body by cutting the respective section of the glass-ceramic dental body with diamond-coated tools.

### 2. Examples

### 2.1 Preparation of glass powders and glass-ceramic dental bodies

To obtain a glass powder, the individual raw materials for the glass powder were weighted, combined and homogenized in a laboratory speed mixer. For example, for preparing glass powders GP4, GP10 and GP 11 (cf. glass ceramic dental body of example 5), the following raw materials were used: quartz powder (SiO₂), lithium carbonate (Li₂CO₃), potassium carbonate (K₂CO₃), aluminum hydroxy hydrate (AIOOH × H₂O), aluminum phosphate (Al(PO₃)₃), magnesium carbonate (MgCO₃), calcium carbonate (CaCOs), strontium carbonate (SrCOs), zirconium oxide (ZrO₂), zinc oxide (ZnO), lanthanum oxide (La₂O₃).

Subsequently, the raw materials were melted at a temperature of 1550 °C for 1 hour. The melt was shock frozen in water to obtain a glass. The glass was dried at 150 °C for 1 hour in an oven and then ground in a laboratory mill to obtain a glass powder. A powder fraction having a particle size of <45 µm were separated and used for preparing a molded body of glass powders.

For preparing a multi-layered molded powder body (three layers), a first powder was introduced in a mold and flattened with a stamp (only stamp weight without additional external pressure). Subsequently, a second powder and a third powder (in that order) was introduced into the mold and flattened with the stamp. Afterwards, the layered powders were pressed with a pressure of 10 bar to obtain the molded powder body. The molded body was removed from the mold by turning the mold 180° and pushing the molded body out of the mold by applying a pressure in the opposite direction.

The molded powder body was then fully sintered under vacuum at 880 °C in a sintering furnace (Programat P500). The molded powder body was first inserted in the sintering furnace (preheated to 400 °C) followed by reducing the pressure in the furnace to < 0.1 MPa. The temperature was increased in a heating step with a heating rate of 10 K/min up to a temperature of 600 °C which was hold for 30 minutes, followed by a heating step with a heating rate of 10 K/min up to a temperature of 880 °C which was likewise hold for 30 minutes. Afterwards, the heating elements of the furnace were deactivated and the pressure was brought to ambient pressure. The fully sintered glass ceramic body (cf. section 2.3: glass ceramic dental body) was removed from the furnace at a temperature of about 500 °C.

### 2.2 Glass ceramic dental bodies

The following tables show the chemical composition of glass powders, the structure and the properties of examples for glass-ceramic dental bodies according to embodiments of the invention. The abbreviation "s.s." in connection with a crystal phase means "solid solution".

### 2.1.2 Example 1

**Table 1.1: example 1**

| **Structure** | **Sections** | **Glass powder** | **Gradient** | **Crystal phases*** |
|---|---|---|---|---|
| | | | Biaxial flexural strength [MPa] | |
| Multi-layer (3 layers) | Section A (top layer) | GP3 | 109 | Li₂Si₂O₅; quartz; Enstatite; Li₃PO₄ |
| | Section B (intermediate layer) | GP2 | 180 | Li₂Si₂O₅; quartz; quartz s.s., Li₃PO₄ |
| | Section C (bottom layer) | GP1 | 188 | Li₂Si₂O₅; quartz s.s; Li₃PO₄ |

| | | | | |
|---|---|---|---|---|
| * the first crystal phase indicated in each field represents the main crystal phase of the section. | | | | |

**Table 1.2: glass powders used for preparing example 1**

| **GP1** | | **GP2** | | **GP3** | |
|---|---|---|---|---|---|
| Component | Weight [wt.%] | Component | Weight [wt.%] | Component | Weight [wt.%] |
| SiO₂ | 71.5 | SiO₂ | 72.5 | SiO₂ | 73.6 |
| Li₂O | 10.1 | Li₂O | 10.2 | Li₂O | 10.5 |
| K₂O | 3.0 | K₂O | 3.0 | K₂O | 3.0 |
| MgO | 4.0 | MgO | 4.0 | MgO | 4.1 |
| SrO | 2.0 | SrO | 2.0 | SrO | 2.0 |
| Al₂O₃ | 2.7 | Al₂O₃ | 1.8 | P₂O₅ | 3.8 |
| P₂O₅ | 3.7 | P₂O₅ | 3.8 | ZrO₂ | 3.0 |
| ZrO₂ | 3.0 | ZrO₂ | 3.0 | | |

### 2.2.2 Example 2

**Table 2.1: example 2**

| **Structure** | **Sections** | **Glass powder** | **Gradients** | | **Crystal phases*** |
|---|---|---|---|---|---|
| | | | Fracture toughness (K_{IC}) [MPa* m^{-1/2}] | Biaxial flexural strength [MPa] | |
| Multi-layer (3 layers) | Section A (top layer) | GP5 | 1.7 | 205 | Li₂Si₂O₅; Li₃PO₄ |
| | Section B (intermed iate layer) | GP4:GP5 (50:50) | 2.1 | 414 | Li₂Si₂O₅; quartz s.s.; Li₃PO₄ |
| | Section C (bottom layer) | GP4 | 2.3 | 426 | Li₂Si₂O₅; quartz s.s.; Li₃PO₄ |

| | | | | | |
|---|---|---|---|---|---|
| * the first crystal phase indicated in each field represents the main crystal phase of the section. | | | | | |

**Table 2.2: glass powders used for preparing example 2**

| **GP4** | | **GP4:GP5** | | **GP5** | |
|---|---|---|---|---|---|
| Component | Weight [wt.%] | Component | Weight [wt.%] | Component | Weight [wt.%] |
| SiO₂ | 71.4 | SiO₂ | 69.5 | SiO₂ | 67.5 |
| Li₂O | 11.8 | Li₂O | 12.9 | Li₂O | 14.0 |
| K₂O | 2.5 | K₂O | 3.1 | K₂O | 3.7 |
| MgO | 1.1 | MgO | 0.5 | Al₂O₃ | 3.2 |
| CaO | 1.5 | CaO | 0.7 | P₂O₅ | 3.6 |
| SrO | 2.7 | SrO | 1.4 | ZrO₂ | 8.0 |
| Al₂O₃ | 2.7 | Al₂O₃ | 3.0 | | |
| P₂O₅ | 3.8 | P₂O₅ | 3.7 | | |
| ZrO₂ | 1.1 | ZrO₂ | 4.5 | | |
| ZnO | 1.4 | ZnO | 0.7 | | |

### 2.3.2 Example 3

**Table 3.1: example 3**

| **Structure** | **Sections** | **Glass powder** | **Gradients** | | **Crystal phases*** |
|---|---|---|---|---|---|
| | | | Fracture toughness (K_{IC}) [MPa* m^{-1/2}] | Biaxial flexural strength [MPa] | |
| Multi-layer (3 layers) | Section A (top layer) | GP6 | 0.9 | 129 | Glass (no crystal phase) |
| | Section B (intermed iate layer) | GP4:GP6 (80:20) | 1.7 | 182 | Li₂Si₂O₅; quartz s.s.; Li₃PO₄, Ca₅(PO₄)₃F |
| | Section C (bottom layer) | GP4 | 2.3 | 426 | Li₂Si₂O₅; quartz s.s.; Li₃PO₄ |

| | | | | | |
|---|---|---|---|---|---|
| * the first crystal phase indicated in each field represents the main crystal phase of the section. | | | | | |

**Table 3.2: glass powders used for preparing example 3**

| **GP4** | | **GP4:GP6** | | **GP6** | |
|---|---|---|---|---|---|
| Component | Weight [wt.%] | Component | Weight [wt.%] | Component | Weight [wt.%] |
| SiO₂ | 71.4 | SiO₂ | 71.2 | SiO₂ | 70.8 |
| Li₂O | 11.8 | Li₂O | 9.5 | Li₂O | 0.4 |
| K₂O | 2.5 | K₂O | 3.3 | K₂O | 6.3 |
| CaO | 1.5 | CaO | 1.5 | CaO | 1.8 |
| SrO | 2.8 | SrO | 2.6 | SrO | 1.9 |
| Al₂O₃ | 2.7 | Al₂O₃ | 3.7 | Al₂O₃ | 7.7 |
| ZnO | 1.4 | ZnO | 1.7 | ZnO | 2.7 |
| B₂O₃ | 0.0 | B₂O₃ | 0.3 | B₂O₃ | 1.5 |
| CeO₂ | 0.0 | CeO₂ | 0.1 | CeO₂ | 0.5 |
| F | 0.0 | F | 0.1 | F | 0.6 |
| Na₂O | 0.0 | Na₂O | 1.2 | Na₂O | 5.8 |
| MgO | 1.0 | MgO | 0.9 | MgO | 0.0 |
| P₂O₅ | 3.8 | P₂O₅ | 3.0 | P₂O₅ | 0.0 |
| ZrO₂ | 1.1 | ZrO₂ | 0.9 | ZrO₂ | 0.0 |

### 2.4.2 Example 4

**Table 4.1: example 4**

| **Structure** | **Sections** | **Glass powder** | **Gradients** | | | **Crystal phases*** |
|---|---|---|---|---|---|---|
| | | | Contrast ratio [%] | Fracture toughness (K_{IC}) [MPa* m^{-1/2}] | Biaxial flexural strength [MPa] | |
| Multi-layer (3 layers) | Section A (top layer) | GP9 | 20.77 | 0.8 | 181 | Glass (no crystal phase) |
| | Section B (intermediate layer) | GP8 | | | | Li₂Si₂O₅; Li₂SiO₃; Li₃PO₄ |
| | Section C (bottom layer) | GP7 | 68.6 | 2.5 | 401 | Li₂Si₂O₅; Li₃PO₄ |

| | | | | | | |
|---|---|---|---|---|---|---|
| * the first crystal phase indicated in each field represents the main crystal phase of the section. | | | | | | |

**Table 4.2: glass powders used for preparing example 4**

| **GP7** | | **GP8** | | **GP9** | |
|---|---|---|---|---|---|
| Component | Weight [wt.%] | Component | Weight [wt.%] | Component | Weight [wt.%] |
| SiO₂ | 71.5 | SiO₂ | 63.3 | SiO₂ | 68.9 |
| Li₂O | 14.3 | Li₂O | 28.0 | Na₂O | 1.5 |
| K₂O | 4.0 | K₂O | 2.5 | K₂O | 13.0 |
| Al₂O₃ | 3.0 | MgO | 0.2 | Al₂O₃ | 8.6 |
| P₂O₅ | 3.2 | SrO | 0.8 | CaO | 7.7 |
| Y₂O₃ | 4.0 | Al₂O₃ | 1.1 | CeO₂ | 0.3 |
| | | P₂O₅ | 1.7 | | |
| | | La₂O₃ | 0.1 | | |
| | | CeO₂ | 0.2 | | |
| | | Tb₄O₇ | 0.1 | | |
| | | ZrO₂ | 0.3 | | |
| | | ZnO | 1.7 | | |

### 2.5.2 Example 5

**Table 5.1: example 5**

| **Structure** | **Sections** | **Glass powder** | **Gradients** | | **Crystal phases*** |
|---|---|---|---|---|---|
| | | | Fracture toughness (K_{IC}) [MPa* m^{-1/2}] | Biaxial flexural strength [MPa] | |
| Multi-layer (3 layers) | Section A (top layer) | GP11 | 1.9 | 245 | quartz s.s.; Li₂Si₂O₅; Li₃PO₄ |
| | Section B (intermediate layer) | GP10 | 2.1 | 401 | quartz s.s.; Li₂Si₂O₅; Li₃PO₄ |
| | Section C (bottom layer) | GP4 | 2.3 | 426 | Li₂Si₂O₅; quartz s.s.; Li₃PO₄ |

| | | | | | |
|---|---|---|---|---|---|
| * the first crystal phase indicated in each field represents the main crystal phase of the section. | | | | | |

**Table 5.2: glass powders used for preparing example 5**

| **GP4** | | **GP10** | | **GP11** | |
|---|---|---|---|---|---|
| Component | Weight [wt.%] | Component | Weight [wt.%] | Component | Weight [wt.%] |
| SiO₂ | 71.3 | SiO₂ | 74.4 | SiO₂ | 76.4 |
| Li₂O | 11.8 | Li₂O | 9.2 | Li₂O | 7.6 |
| K₂O | 2.5 | K₂O | 2.4 | K₂O | 2.4 |
| MgO | 1.1 | MgO | 1.0 | MgO | 1.0 |
| CaO | 1.5 | CaO | 1.5 | CaO | 1.4 |
| SrO | 2.8 | SrO | 2.7 | SrO | 2.6 |
| Al₂O₃ | 2.7 | Al₂O₃ | 2.6 | Al₂O₃ | 2.6 |
| P₂O₅ | 3.8 | P₂O₅ | 3.7 | P₂O₅ | 3.6 |
| ZrO₂ | 1.1 | ZrO₂ | 1.1 | ZrO₂ | 1.0 |
| ZnO | 1.4 | ZnO | 1.4 | ZnO | 1.4 |

### 2.6.2 Example 6

**Table 6.1: example 6**

| **Structure** | **Sections** | **Glass powder** | **Gradient** | **Crystal phases*** |
|---|---|---|---|---|
| | | | CTE [ppm/K] | |
| Multi-layer (3 layers) | Section A (top layer) | GP14 | 6.19 | quartz s.s.; Li₂Si₂O₅; Li₃PO₄ |
| | Section B (intermediate layer) | GP13 | 7.27 | quartz s.s.; Li₂Si₂O₅; Li₃PO₄ |
| | Section C (bottom layer) | GP12 | 8.64 | Li₂Si₂O₅; α-quartz s.s.; Li₃PO₄ |

| | | | | |
|---|---|---|---|---|
| * the first crystal phase indicated in each field represents the main crystal phase of the section. | | | | |

**Table 6.2: glass powders used for preparing example 6**

| **GP12** | | **GP13** | | **GP14** | |
|---|---|---|---|---|---|
| Component | Weight [wt.%] | Component | Weight [wt.%] | Component | Weight [wt.%] |
| SiO₂ | 69.5 | SiO₂ | 69.0 | SiO₂ | 68.2 |
| Li₂O | 10.2 | Li₂O | 10.1 | Li₂O | 10.0 |
| K₂O | 2.4 | K₂O | 2.4 | K₂O | 2.4 |
| MgO | 3.8 | MgO | 3.7 | MgO | 3.7 |
| CaO | 0.4 | CaO | 0.4 | CaO | 0.4 |
| SrO | 1.8 | SrO | 1.8 | SrO | 1.8 |
| Al₂O₃ | 5.3 | Al₂O₃ | 6.2 | Al₂O₃ | 7.1 |
| P₂O₅ | 3.7 | P₂O₅ | 3.6 | P₂O₅ | 3.6 |
| La₂O₃ | 0.5 | La₂O₃ | 0.5 | La₂O₃ | 0.5 |
| ZrO₂ | 2.4 | ZrO₂ | 2.3 | ZrO₂ | 2.3 |
| ZnO | 0.2 | ZnO | 0.2 | ZnO | 0.2 |

### 2.7.2 Example 7

**Table 7.1: example 7**

| **Structure** | **Sections** | **Glass powder** | **Gradient** | **Crystal phases*** |
|---|---|---|---|---|
| | | | Contrast ratio [%] | |
| Multi-layer (3 layers) | Section A (top layer) | GP16 | 64.63 | quartz s.s.; Li₂Si₂O₅; Li₃PO₄ |
| | Section B (intermed iate layer) | GP15 | 70.94 | quartz s.s.; Li₂Si₂O₅; Li₂O·Al₂O₃·7.5SiO₂ (Spodumene s.s.); Li₃PO₄ |
| | Section C (bottom layer) | GP14 | 88.49 | quartz s.s.; Li₂Si₂O₅; Li₂O·Al₂O₃·7.5SiO₂ (Spodumene s.s.); Li₃PO₄ |

| | | | | |
|---|---|---|---|---|
| * the first crystal phase indicated in each field represents the main crystal phase of the section. | | | | |

**Table 7.2: glass powders used for preparing example 7**

| **GP14** | | **GP15** | | **GP16** | |
|---|---|---|---|---|---|
| Component | Weight [wt.%] | Component | Weight [wt.%] | Component | Weight [wt.%] |
| SiO₂ | 68.2 | SiO₂ | 68.8 | SiO₂ | 69.1 |
| Li₂O | 10.0 | Li₂O | 10.1 | Li₂O | 10.1 |
| K₂O | 2.4 | K₂O | 1.7 | K₂O | 1.1 |
| MgO | 3.7 | MgO | 3.7 | MgO | 3.7 |
| CaO | 0.4 | CaO | 0.4 | CaO | 0.4 |
| SrO | 1.8 | SrO | 1.8 | SrO | 1.8 |
| Al₂O₃ | 7.1 | Al₂O₃ | 7.1 | Al₂O₃ | 7.2 |
| P₂O₅ | 3.6 | P₂O₅ | 3.6 | P₂O₅ | 3.7 |
| La₂O₃ | 0.5 | La₂O₃ | 0.5 | La₂O₃ | 0.5 |
| ZrO₂ | 2.3 | ZrO₂ | 2.3 | ZrO₂ | 2.4 |
| ZnO | 0.2 | ZnO | 0.2 | ZnO | 0.2 |

### 2.8.2 Example 8

**Table 8.1: example 8**

| **Structure** | **Sections** | **Glass powder** | **Gradients** | | **Crystal phases*** |
|---|---|---|---|---|---|
| | | | Fracture toughness (K_{IC}) [MPa* m^{-1/2}] | Biaxial flexural strength [MPa] | |
| Multi-layer (3 layers) | Section A (top layer) | GP19 | 0.8 | 162 | Ca₅(PO₄)₃F |
| | Section B (intermed iate layer) | GP18 | | | Li₂Si₂O₅; quartz s.s.; Li₃PO₄ |
| | Section C (bottom layer) | GP17 | 1.6 | 288 | Li₂Si₂O₅; quartz s.s.; Li₃PO₄ |

| | | | | | |
|---|---|---|---|---|---|
| * the first crystal phase indicated in each field represents the main crystal phase of the section. | | | | | |

**Table 8.2: glass powders used for preparing example 8**

| **GP17** | | **GP18** | | **GP19** | |
|---|---|---|---|---|---|
| Component | Weight [wt.%] | Component | Weight [wt.%] | Component | Weight [wt.%] |
| SiO₂ | 71.0 | SiO₂ | 70.9 | SiO₂ | 59.3 |
| Li₂O | 10.1 | Li₂O | 9.9 | Li₂O | 0.4 |
| K₂O | 3.0 | K₂O | 3.0 | Na₂O | 5.9 |
| MgO | 3.8 | MgO | 3.8 | K₂O | 6.4 |
| CaO | 0.4 | CaO | 0.3 | CaO | 7.2 |
| SrO | 1.9 | SrO | 1.8 | SrO | 1.9 |
| Al₂O₃ | 3.2 | Al₂O₃ | 3.2 | B₂O₃ | 1.5 |
| P₂O₅ | 3.7 | P₂O₅ | 4.2 | Al₂O₃ | 5.8 |
| La₂O₃ | 0.5 | La₂O₃ | 0.5 | P₂O₅ | 4.5 |
| ZrO₂ | 2.4 | ZrO₂ | 2.4 | F | 2.1 |
| ZnO | 0.2 | ZnO | 0.2 | TiO₂ | 1.3 |
| | | | | ZrO₂ | 0.5 |
| | | | | CeO₂ | 0.5 |
| | | | | ZnO | 2.7 |

### 2.9.2 Example 9

**Table 9.1: example 9**

| **Structure** | **Sections** | **Glass powder** | **Gradient** | **Crystal phases*** |
|---|---|---|---|---|
| | | | CTE [ppm/K] | |
| Multi-layer (3 layers) | Section A (top layer) | GP22 | 12.72 | quartz; Li₂Si₂O₅; Li₃PO₄ |
| | Section B (intermed iate layer) | GP21 | 12.03 | quartz; Li₂Si₂O₅; Li₃PO₄ |
| | Section C (bottom layer) | GP20 | 11.27 | Li₂Si₂O₅; quartz; Li₃PO₄ |

| | | | | |
|---|---|---|---|---|
| * the first crystal phase indicated in each field represents the main crystal phase of the section. | | | | |

**Table 9.2: glass powders used for preparing example 9**

| **GP20** | | **GP21** | | **GP22** | |
|---|---|---|---|---|---|
| Component | Weight [wt.%] | Component | Weight [wt.%] | Component | Weight [wt.%] |
| SiO₂ | 73.9 | SiO₂ | 77.4 | SiO₂ | 79.7 |
| Li₂O | 12.3 | Li₂O | 9.6 | Li₂O | 7.9 |
| K₂O | 3.3 | K₂O | 3.2 | K₂O | 3.2 |
| MgO | 4.3 | MgO | 4.3 | MgO | 4.2 |
| SrO | 2.2 | SrO | 2.1 | SrO | 2.1 |
| P₂O₅ | 4.0 | P₂O₅ | 3.4 | P₂O₅ | 2.9 |

### 2.10.2 Example 10

**Table 10.1: example 10**

| **Structure** | **Sections** | **Glass powder** | **Gradients** | | **Crystal phases*** |
|---|---|---|---|---|---|
| | | | Fracture toughness (K_{IC}) [MPa* m^{-1/2}] | Biaxial flexural strength [MPa] | |
| Multi-layer (3 layers) | Section A (top layer) | GP24 | 2.7 | 269 | Li₂Si₂O₅; Li₂SiO₃ |
| | Section B (intermed iate layer) | GP24:GP23 (50:50) | 2.9 | 286 | Li₂Si₂O₅; LiSiO₃ |
| | Section C (bottom layer) | GP23 | 3.1 | 299 | Li₂Si₂O₅; |

| | | | | | |
|---|---|---|---|---|---|
| * the first crystal phase indicated in each field represents the main crystal phase of the section. | | | | | |

**Table 10.2: example 10**

| **GP24** | | **GP24:GP23** | | **GP23** | |
|---|---|---|---|---|---|
| Component | Weight [wt.%] | Component | Weight [wt.%] | Component | Weight [wt.%] |
| SiO₂ | 76.7 | SiO₂ | 77.9 | SiO₂ | 79.4 |
| Li₂O | 16.0 | Li₂O | 16.3 | Li₂O | 16.5 |
| K₂O | 3.6 | K₂O | 2.7 | K₂O | 1.8 |
| Al₂O₃ | 3.7 | CuO | 0.1 | CuO | 0.1 |
| | | Fe | 0.1 | Fe | 0.2 |
| | | Al₂O₃ | 2.9 | Al₂O₃ | 2.0 |

## Claims

1. A glass-ceramic dental body comprising three consecutive sections:
a section A,
a section B, and
a section C,
wherein each one of the sections has a chemical composition that differs from a chemical composition of the other sections, and
wherein the glass ceramic-dental body is **characterized by** one or more gradients of a mechanical property, an optical property, and/or a thermal property in a direction from section C to section A.

2. The glass-ceramic dental body according to claim 1, being **characterized by**
a gradient of a biaxial flexural strength, wherein the biaxial flexural strength decreases from section C to section A; and/or
a gradient of a fracture toughness (K_{IC}), wherein the fracture toughness (K_{IC}) decreases from section C to section A.

3. The glass-ceramic dental body according to claim 1 or claim 2, being **characterized by** a gradient of a contrast ratio, wherein the contrast ratio decreases from section C to section A.

4. The glass-ceramic dental body according to any one of claims 1 to 3, being **characterized by** a gradient of a coefficient of the thermal expansion, wherein the coefficient of the thermal expansion decreases or increases from section C to section A.

5. The glass-ceramic dental body according to any one of claims 1 to 4, wherein a main crystal phase of each one of section C, section B and section A is the same.

6. The glass-ceramic dental body according to claim 5, wherein a content of the main crystal phase decreases in a direction from section C to section A.

7. The glass-ceramic dental body according to any one of claims 1 to 4, wherein a main crystal phase of section C is different to a main crystal phase of section A.

8. The glass-ceramic dental body according to claim 7, wherein a content of the crystal phase, which is the main crystal phase of section C, decreases in a direction from section C to section A, or
wherein a content of the crystal phase, which is the main crystal phase of section A, decreases in a direction from section A to section C.

9. The glass-ceramic dental body according to any one of claims 1 to 4, wherein section A is composed of a glass, and wherein section B and section C are composed of a glass-ceramic.

10. The glass-ceramic dental body according to any one of the preceding claims, wherein section C has a main crystal phase, and a content of this crystal phase decreases from section C to section A.

11. The glass-ceramic dental body according to any one of the preceding claims, wherein a main crystal phase of section C is lithium disilicate, a quartz, or a stoichiometric or non-stoichiometric lithium alumosilicate (LAS).

12. The glass-ceramic dental body according to claim 11, wherein the main crystal phase of section C is lithium disilicate.

13. The glass-ceramic dental body according to any one of the preceding claims, wherein a main crystal phase of section C is not lithium metasilicate.

14. The glass-ceramic dental body according to any one of claims 1 to 4, 7, 8, 10 to 13, wherein a main crystal phase of section A is an apatite, a quartz, or a stoichiometric or non-stoichiometric lithium alumosilicate (LAS).

15. The glass-ceramic dental body according to claim 14, wherein the main crystal phase of section A is a quartz.

16. The glass-ceramic dental body according to any one of the preceding claims, wherein the glass-ceramic dental body is a dental blank, like a dental mill blank or a dental press blank, and optionally wherein:
section A forms a top section of the dental blank,
section B forms an intermediate section of the dental blank, and
section C forms a bottom section of the dental blank.

17. The glass-ceramic dental body according to any one of claims 1 to 15, wherein the glass-ceramic dental body is a dental restoration, and optionally wherein:
section A forms at least a part of an incisal zone,
section B forms at least a part of a transitional zone between an incisal zone and a dentin zone,and
section C forms at least a part of a dentin zone.

18. The glass-ceramic dental body according to any one of the preceding claims, having a multi-layer structure, wherein section A is a layer A, section C is a layer C, and section B is formed by at least one intermediate layer between layer A and layer C, and each one of the layers has a chemical composition that differs from a chemical composition of the other layers, or
wherein section A and section C have a homogenous chemical composition, and section B has a chemical composition that gradually changes in a direction from section C to section A.

19. A process for preparing a glass-ceramic dental body according to any one of the preceding claims, the process comprising the steps of:
- providing two or more powders selected from glass powders, glass-ceramic powders and mixtures thereof;
- preparing a shaped body from the powders; and
- subjecting the shaped body to a heat treatment to obtain a glass-ceramic dental body.
